(19) Europäisches Patentamt
European Patent Office
Office européen des brevets

(11) EP 3 438 088 A1

(12) EUROPEAN PATENT APPLICATION
published in accordance with Art. 153(4) EPC

(43) Date of publication:
06.02.2019 Bulletin 2019/06

(21) Application number: 17775419.9

(22) Date of filing: 30.03.2017

(51) Int Cl.:
*C07C 239/08* (2006.01) *A61K 31/166* (2006.01)
*A61P 31/04* (2006.01) *A61P 43/00* (2006.01)

(86) International application number:
PCT/JP2017/013290

(87) International publication number:
WO 2017/170885 (05.10.2017 Gazette 2017/40)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
MA MD

(30) Priority: 31.03.2016 JP 2016070504

(71) Applicant: Toyama Chemical Co., Ltd.
Shinjuku-ku
Tokyo 160-0023 (JP)

(72) Inventors:
- BABA, Yasutaka
Toyama-shi
Toyama 930-8508 (JP)
- NAGATO, Yusuke
Toyama-shi
Toyama 930-8508 (JP)
- KOSEKI, Yu
Toyama-shi
Toyama 930-8508 (JP)

(74) Representative: Hoffmann Eitle
Patent- und Rechtsanwälte PartmbB
Arabellastraße 30
81925 München (DE)

# (54) CRYSTALS OF NOVEL HYDROXAMIC ACID DERIVATIVE, METHOD FOR PRODUCING SAME, AND PHARMACEUTICAL COMPOSITION

(57) Crystals of (2S)-2-((4-((4-((1S)-1,2-dihydroxyethyl)phenyl)ethynyl)benzoyl)(methyl)amino)-N-hydroxy-N',2-dimethylmalonamide having diffraction peaks at diffraction angles (2θ) of 3.8 ± 0.2°, 7.7 ± 0.2°, and 10.8 ± 0.2°, etc., or 8.2 ± 0.2°, 12.4 ± 0.2°, and 13.3 ± 0.2°, etc., in powder X-ray diffraction, a production method thereof, and a pharmaceutical composition.

EP 3 438 088 A1

## Description

Technical Field

The present invention relates to crystals of (2S)-2-((4-((4-((1 S)-1,2-dihydroxyethyl)phenyl)ethynyl)benzoyl)(methyl)amino)-N-hydroxy-N',2-dimethylmalonamide or a hydrate thereof having excellent inhibitory activity against uridyldiphospho-3-O-acyl-N-acetylglucosamine deacetylase (LpxC), and production method thereof. The present invention further relates to a pharmaceutical composition comprising the crystals.

Background Art

LpxC is an enzyme that catalyzes the synthesis of lipid A. The lipid A is a component essential for outer membrane formation and is essential for, for example, the survival of gram-negative bacterial. Thus, a drug inhibiting the activity of LpxC is strongly expected to be able to serve as an antibacterial agent effective for gram-negative bacteria including *Pseudomonas aeruginosa.*

For example, (2S)-2-((4-((4-((1S)-1,2-dihydroxyethyl)phenyl)ethynyl)benzoyl)(met hyl)amino)-N-hydroxy-N',2-dimethylmalonamide (hereinafter, also referred to as compou nd A) having excellent LpxC inhibitory activity is known (Patent Literature 1).

Prior Art Literatures

Patent Literatures

Patent Literature 1: International Publication No. WO 2014/142298 pamphlet

Summary of Invention

Object to be Solved by the Invention

A crystal of a hydrate of compound A (hereinafter, referred to as "type I crystals") produced according to a production method described in Patent Literature 1 has hygroscopic properties and exhibits poor stability. In the case of using compound A as a bulk pharmaceutical, it is strongly desired to provide crystals excellent in stability.

An object of the present invention is to provide novel crystals of compound A or a hydrate thereof which are excellent in stability and useful as a bulk pharmaceutical, a production method thereof, and a pharmaceutical composition.

Means for Solving the Object

Under these circumstances, the present inventors have conducted diligent studies and consequently completed the present invention by finding that a crystal of a hydrate of compound A having diffraction peaks at diffraction angles (2θ) of 3.8 ± 0.2°, 7.7 ± 0.2°, 10.8 ± 0.2°, 12.0 ± 0.2°, and 14.4 ± 0.2° (hereinafter, referred to as "type II crystals") and a crystal of compound A having diffraction peaks at diffraction angles (2θ) of 8.2 ± 0.2°, 12.4 ± 0.2°, 13.3 ± 0.2°, 15.2 ± 0.2°, and 16.2 ± 0.2° (hereinafter, referred to as "type III crystals") in powder X-ray diffraction are excellent in stability, easily handleable, and excellent as a bulk pharmaceutical.

Moreover, the present inventors have also found that type II crystal and type III crystal could be selectively produced by selecting a suitable solvent, thereby completing the present invention.

The present invention provides the following subject matters.

[1] A crystal of a hydrate of (2S)-2-((4-((4-((1S)-1,2-dihydroxyethyl)phenyl)ethynyl)benzoyl)(methyl)amino)-N-hydroxy-N',2-dimethylmalonamide having diffraction peaks at diffraction angles (2θ) of 3.8 ± 0.2°, 7.7 ± 0.2°, 10.8 ± 0.2°, 12.0 ± 0.2°, and 14.4 ± 0.2° in powder X-ray diffraction.

[2] A crystal of a hydrate of (2S)-2-((4-((4-((1S)-1,2-dihydroxyethyl)phenyl)ethynyl)benzoyl)(methyl)amino)-N-hydroxy-N',2-dimethylmalonamide having diffraction peaks at diffraction angles (2θ) of 3.8 ± 0.2°, 7.7 ± 0.2°, 10.8 ± 0.2°, 12.0 ± 0.2°, 14.4 ± 0.2°, 16.3 ± 0.2°, 17.0 ± 0.2°, and 21.8 ± 0.2° in powder X-ray diffraction.

[3]

A crystal of (2S)-2-((4-((4-((1S)-1,2-dihydroxyethyl)phenyl)ethynyl)benzoyl)(methyl)amino)-N-hydroxy-N',2-dimethylmalonamide having diffraction peaks at diffraction angles (2θ) of 8.2 ± 0.2°, 12.4 ± 0.2°, 13.3 ± 0.2°, 15.2 ± 0.2°, and 16.2 ± 0.2° in powder X-ray diffraction.

[4] A crystal of (2S)-2-((4-((4-((1S)-1,2-dihydroxyethyl)phenyl)ethynyl)benzoyl)(methyl)amino)-N-hydroxy-N',2-dimethylmalonamide having diffraction peaks at diffraction angles (2θ) of 8.2 ± 0.2°, 12.4 ± 0.2°, 13.3 ± 0.2°, 15.2 ± 0.2°, 16.2 ± 0.2°, 19.0 ± 0.2°, 20.2 ± 0.2°, and 22.8 ± 0.2° in powder X-ray diffraction.
[5] A pharmaceutical composition which comprises the crystal according to any one of [1] to [4].
[6] A method for producing the crystal according to [1] or [2], comprising a step of stirring a mixture containing (1) Compound A, (2) water, and (3) an organic solvent, wherein the organic solvent is one or two or more selected from alcohols, ethers, ketones, and nitriles.
[7] The production method according to [6], wherein the alcohols are methanol, ethanol and 2-propanol, the ether is tetrahydrofuran, the ketone is acetone, and the nitrile is acetonitrile.
[8] The production method according to [6] or [7], wherein the Compound A is type I crystal.
[9] A method for producing the crystal according to [3] or [4], comprising a step of stirring a mixture containing (1) Compound A and (2) an organic solvent, in the absence of water, wherein the organic solvent is one or two or more selected from aliphatic hydrocarbons, alcohols, ethers, ketones, esters, sulfoxides, nitriles, and amides.
[10] The production method according to [9], wherein the aliphatic hydrocarbon is heptane, the alcohols are methanol, ethanol and 2-propanol, the ether is tetrahydrofuran, the ketone is acetone, the ester is ethyl acetate, the sulfoxide is dimethyl sulfoxide, the nitrile is acetonitrile, and the amide is dimethylacetamide.
[11] The production method according to [9] or [10], wherein the Compound A is the crystal according to [1] or [2].

The present invention further provides the following.

[A] A method for producing the crystal according to [1] or [2], comprising a step of subjecting a compound represented by the formula [1]:

[Formula 1]

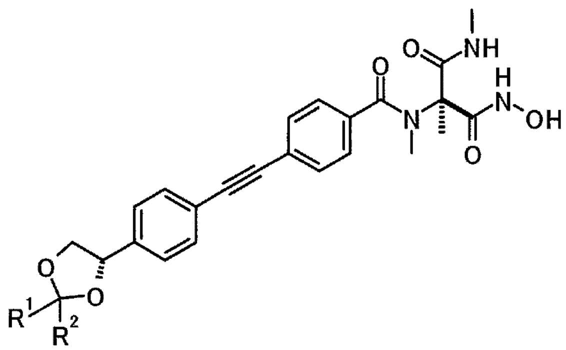

[1]

wherein $R^1$ represents a $C_{1-3}$ alkyl group; and $R^2$ represents a $C_{1-3}$ alkyl group,
to a deprotection reaction to produce Compound A, and then stirring the Compound A in a mixture of water and an organic solvent, without isolating the Compound A, wherein the organic solvent is one or two or more selected from alcohols, ethers, ketones, and nitriles.
[B]
The production method according to [A], wherein $R^1$ is a methyl group, and $R^2$ is a methyl group.
[C] The production method according to [A] or [B], wherein the alcohols are methanol, ethanol and 2-propanol, the ether is tetrahydrofuran, the ketone is acetone, and the nitrile is acetonitrile.

Advantageous Effects of Invention

The type II crystals of the present invention are excellent in stability, easily handleable, and useful as a bulk pharmaceutical.
The type III crystals of the present invention are excellent in stability, easily handleable, and useful as a bulk pharmaceutical. Also, the type III crystals are excellent in solubility at a low temperature when a solubilizing agent such as cyclodextrin is used.
Since type II crystal or type III crystal can be selectively produced according to the production method of the present invention, the present production method is useful as an industrial production method.

Brief Description of Drawings

Figure 1 is a diagram showing one example of an infrared absorption spectrum (ATR method) of type I crystals of a hydrate of compound A (Reference Example 1).

Figure 2 is a diagram showing one example of a powder X-ray diffraction pattern of the type I crystals of a hydrate of compound A (Reference Example 1).

Figure 3 is a diagram showing one example of an infrared absorption spectrum (ATR method) of type II crystals of a hydrate of compound A (Example 1).

Figure 4 is a diagram showing one example of a powder X-ray diffraction pattern of the type II crystals of a hydrate of compound A (Example 1).

Figure 5 is a diagram showing one example of an infrared absorption spectrum (ATR method) of type II crystals of a hydrate of compound A (Example 3).

Figure 6 is a diagram showing one example of a powder X-ray diffraction pattern of the type II crystals of a hydrate of compound A (Example 3).

Figure 7 is a diagram showing one example of an infrared absorption spectrum (ATR method) of type III crystals of compound A (Example 4).

Figure 8 is a diagram showing one example of a powder X-ray diffraction pattern of the type III crystals of compound A (Example 4).

Figure 9 is a diagram showing one example of an infrared absorption spectrum (ATR method) of type III crystals of compound A (Example 5).

Figure 10 is a diagram showing one example of a powder X-ray diffraction pattern of the type III crystals of compound A (Example 5).

Embodiments for Carrying out the Invention

Hereinafter, the present invention will be described in detail.

In the present invention, % is % by mass unless otherwise specified.

In the present invention, each term has the following meaning unless otherwise specified.

The $C_{1-3}$ alkyl group means a methyl, ethyl, propyl or isopropyl group.

Examples of aliphatic hydrocarbons include pentane, hexane, heptane, and cyclohexane.

Examples of alcohols include methanol, ethanol, propanol, 2-propanol, butanol, and 2-methyl-2-propanol.

Examples of ethers include methyl tert-butyl ether, dioxane, tetrahydrofuran, 2-methyltetrahydrofuran, cyclopentylmethylether, and anisole.

Examples of ketones include acetone, 2-butanone, and 4-methyl-2-pentanone.

Examples of esters include methyl acetate, ethyl acetate, propyl acetate, and butyl acetate.

Examples of sulfoxides include dimethyl sulfoxide, and sulfolane.

Examples of nitriles include acetonitrile and propionitrile.

Examples of amides include N,N-dimethylformamide, N,N-dimethylacetamide, and 1-methyl-2-pyrrolidone.

In general, a diffraction angle (2θ) in powder X-ray diffraction may have an error within the range of ±0.2°. Thus, the term "diffraction angle (2θ) of X°" described in the present invention means "diffraction angle (2θ) of ((X - 0.2) to (X + 0.2))°" unless otherwise specified. Accordingly, not only crystals having perfectly matching diffraction angles in powder X-ray diffraction but crystals having matching diffraction angles within the error range of ±0.2° are included in the present invention.

Besides, it has been known that the relative intensity in the powder X-ray diffraction fluctuates depending on crystal orientation, the size of a particle, crystallinity, measurement conditions, etc. Thus, it should not be rigidly understood.

In general, the value of a wave number ($cm^{-1}$) in an infrared absorption spectrum (ATR method) may have an error within the range of ±2 $cm^{-1}$. Thus, the term "wave number of Y $cm^{-1}$" described in the present invention means "wave number of ((Y - 2) to (Y + 2)) $cm^{-1}$" unless otherwise specified. Accordingly, not only crystals having perfectly matching wave numbers of absorption peaks in an infrared absorption spectrum (ATR method) but crystals having matching wave numbers of absorption peaks within the error range of ±2 $cm^{-1}$ are included in the present invention.

The type II crystals of the present invention are characterized by diffraction peaks in powder X-ray diffraction.

The type II crystals of the present invention have diffraction peaks at diffraction angles (2θ) of 3.8 ± 0.2°, 7.7 ± 0.2°, 10.8 ± 0.2°, 12.0 ± 0.2°, and 14.4 ± 0.2° in powder X-ray diffraction.

The type II crystals of the present invention further have diffraction peaks at 16.3 ± 0.2°, 17.0 ± 0.2°, and 21.8 ± 0.2° in powder X-ray diffraction.

The type II crystals of the present invention are also characterized by absorption peaks in an infrared absorption spectrum (ATR method).

The type II crystals of the present invention have absorption peaks at wave numbers of 1475 ± 2 $cm^{-1}$, 1606 ± 2 $cm^{-1}$, 1683 ± 2 $cm^{-1}$, 3134 ± 2 $cm^{-1}$, and 3365 ± 2 $cm^{-1}$ in an infrared absorption spectrum (ATR method).

The type III crystals of the present invention are characterized by diffraction peaks in powder X-ray diffraction.

The type III crystals of the present invention have diffraction peaks at diffraction angles (2θ) of 8.2 ± 0.2°, 12.4 ± 0.2°, 13.3 ± 0.2°, 15.2 ± 0.2°, and 16.2 ± 0.2° in powder X-ray diffraction.

The type III crystals of the present invention further have diffraction peaks at 19.0 ± 0.2°, 20.2 ± 0.2°, and 22.8 ± 0.2° in powder X-ray diffraction.

The type III crystals of the present invention are also characterized by absorption peaks in an infrared absorption spectrum (ATR method).

The type III crystals of the present invention have absorption peaks at wave numbers of 1484 ± 2 $cm^{-1}$, 1608 ± 2 $cm^{-1}$, 1688 ± 2 $cm^{-1}$, 3288 ± 2 $cm^{-1}$, and 3475 ± 2 $cm^{-1}$ in an infrared absorption spectrum (ATR method).

Next, the type II crystals and the type III crystals of the present invention will be described.

[Type I crystals]

The type I crystals can be produced according to a production method described in Patent Literature 1.

As a result of measuring moisture, the type I crystals were a monohydrate.

[Type II crystals]

The type II crystals can be produced, for example by stirring a mixture containing (1) Compound A, (2) water, and (3) an organic solvent.

Herein, the organic solvent is one or two or more selected from alcohols, ethers, ketones, and nitriles.

Examples of the Compound A used in this step include the type I crystal and the after-mentioned type III crystal. It is preferable to use the type I crystal.

Water may be used in an amount of 2 to 50 times (v/w), and preferably 5 to 20 times (v/w) larger than the amount of the Compound A.

The alcohols used in this step are one or two or more selected from methanol, ethanol, and 2-propanol. Among these, 2-propanol is preferable.

The ethers used in this step are one or two selected from dioxane and tetrahydrofuran, and among these, tetrahydrofuran is preferable.

The ketones used in this step are one or two selected from acetone and 2-butanone, and among these, acetone is preferable.

The nitriles used in this step are one or two selected from acetonitrile and propionitrile, and among these, acetonitrile is preferable.

The organic solvent used in this step is preferably one or two or more selected from alcohols, ethers, and nitriles. Among these, one or two or more selected from methanol, ethanol, 2-propanol, tetrahydrofuran and acetonitrile are more preferable, and acetonitrile is further preferable.

The organic solvent may be used in an amount of 2 to 50 times (v/w), and preferably 5 to 20 times (v/w) larger than that of the Compound A.

The stirring temperature is preferably 0°C to 70°C, and more preferably 0°C to 30°C.

The stirring time is preferably 1 to 48 hours, and more preferably 1 to 24 hours.

Moreover, the type II crystal can also be produced, for example, by the following production method.

[Formula 2]

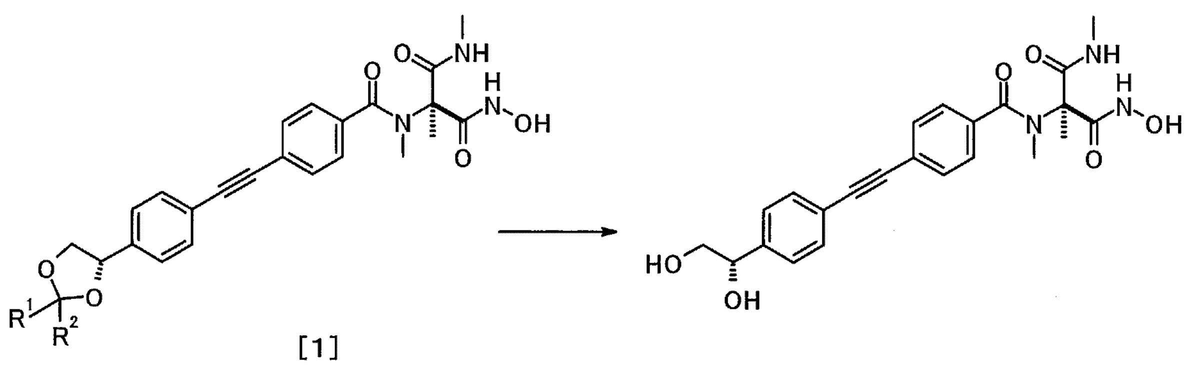

[1]

wherein $R^1$ represents a $C_{1-3}$ alkyl group; and $R^2$ represents a $C_{1-3}$ alkyl group.

The compound represented by the formula [1] can be produced, for example, by the following production example.

In the above formula, $R^1$ represents a $C_{1-3}$ alkyl group. $R^1$ is preferably a methyl group.

$R^2$ represents a $C_{1-3}$ alkyl group. $R^2$ is preferably a methyl group.

More preferably, $R^1$ is a methyl group, and $R^2$ is a methyl group.

The type II crystal can also be produced by deprotecting the compound represented by the formula [1] to produce Compound A, and then stirring a mixture containing the Compound A, water and an organic solvent, without isolating the Compound A. Herein, the organic solvent is one or two or more selected from alcohols, ethers, ketones, and nitriles.

The amount of water used, the type of the organic solvent, the amount of the organic solvent used, the temperature, and the time are the same as those described above.

In this reaction, a seed crystal of the type II crystal is preferably added.

As a result of the measurement of the water content, the type II crystal was found to be a monohydrate.

[Type III crystal]

The type III crystal can be produced, for example, by stirring a mixture containing (1) Compound A and (2) an organic solvent, in the absence of water.

Herein, production in the absence of water means that the crystal is produced without addition of water in the production of the type III crystal. Moreover, water contained in the Compound A or the organic solvent is allowed to be contained in the mixture.

Herein, the organic solvent is one or two or more selected from aliphatic hydrocarbons, alcohols, ethers, ketones, esters, sulfoxides, nitriles, and amides.

Examples of the Compound A used in this step include type I crystal and type II crystal. It is preferable to use the type II crystal.

The aliphatic hydrocarbons used in this step are one or two or more selected from hexane, heptane and cyclohexane, and among these, heptane is preferable.

The alcohols used in this step are one or two or more selected from methanol, ethanol, and 2-propanol. Among these, methanol is preferable.

The ethers used in this step are one or two selected from dioxane and tetrahydrofuran, and among these, tetrahydrofuran is preferable.

The ketones used in this step are one or two selected from acetone and 2-butanone, and among these, acetone is preferable.

The esters used in this step are one or two selected from methyl acetate and ethyl acetate, and among these, ethyl acetate is preferable.

The sulfoxides used in this step are one or two selected from dimethyl sulfoxide and sulfolane, and among these, dimethyl sulfoxide is preferable.

The nitriles used in this step are one or two selected from acetonitrile and propionitrile, and among theses, acetonitrile is preferable.

The amides used in this step are one or two selected from N,N-dimethylformamide and N,N-dimethylacetamide, and among these, N,N-dimethylacetamide is preferable.

The organic solvent used in this step is preferably one or two or more selected from aliphatic hydrocarbons, alcohols, esters, and sulfoxides. Among these, one or two or more selected from heptane, methanol, ethyl acetate and dimethyl sulfoxide are more preferable, and a mixed solvent of heptane, methanol, ethyl acetate and dimethyl sulfoxide is more preferable.

The organic solvent may be used in an amount of 1 to 100 times (v/w), and preferably 5 to 50 times (v/w) larger than that of the Compound A.

The stirring temperature is preferably 0°C to 70°C, and more preferably 0°C to 30°C.

The stirring time is preferably 1 to 8 hours, and more preferably 2 to 4 hours.

In this reaction, a seed crystal of the type III crystal is preferably added.

Moreover, in an industrial production method, the type III crystal can also be produced, for example, by crystallizing Compound A from an organic solvent in the absence of water.

The used organic solvent, the amount of the organic solvent used, the stirring temperature, and the stirring time are the same as those described above.

As a result of the measurement of the water content, the type III crystal was found to be an anhydrate.

In the case of using the type II crystals and the type III crystals of the present invention as a pharmaceutical product, the type II crystals or the type III crystals may be appropriately mixed with pharmaceutical aids, such as an excipient, a carrier, and a diluent, usually used in formulation.

These preparations can be administered orally or parenterally in forms such as tablets, capsules, powders, syrups, granules, pills, suspensions, emulsions, solutions, powder preparations, suppositories, eye drops, nasal drops, ear drops, patches, ointments, lotions, creams, or injections. An administration method, a dose, and the number of doses can be appropriately selected according to the age, body weight, and symptoms of a patient. Usually, the preparations can each be administered at a daily dose of 0.01 to 1000 mg/kg in one portion or several divided portions to an adult by oral or parenteral (e.g., injection, drip infusion, and administration to a rectal site) administration.
Next, the usefulness of the type II crystals and the type III crystals of the present invention will be described with reference to Test Examples below.

Test Example 1 Hygroscopic properties

The type I crystals and the type III crystals were stored under conditions of 25°C and a relative humidity of 97%.
These crystals were sampled over time, and the weights of the crystals were measured to determine the rates of change in weight. The results are shown below.

$$\text{Rate of change in weight (\%)} = ((B - A) / A) \times 100$$

A: Weight of the crystals before the start of the test
B: Weight of the crystals after the start of the test

[Table 1]

| | Rate of change in weight (%) | |
|---|---|---|
| | Type I crystals | Type III crystals |
| 4 weeks | 0.80 | 0.10 |
| 13 weeks | 1.09 | - |
| 16 weeks | - | 0.03 |

The rate of change in the weight of the type I crystals after 13 weeks was 1% or more. On the other hand, the rate of change in the weight of the type III crystals was 0.1% or less.
The type III crystals had low hygroscopic properties.

Test Example 2 Physical stability (1)

The type I crystals and the type II crystals were mixed, suspended in a 40% aqueous acetonitrile solution, and stirred at 25°C.
The mixture was sampled over time, and the crystal form was measured by powder X-ray diffraction.
The results are shown below.

[Table 2]

| | 25°C |
|---|---|
| 0 hr | I + II |
| 0.5 hr | II |
| 1 hr | II |

The type I crystals were transformed to the type II crystals.
The type II crystals were more stable than the type I crystals.

Test Example 3 Physical stability (2)

The type I crystals and the type II crystals were mixed, suspended in acetonitrile, and stirred at 5, 25, or 40°C.
The mixture was sampled over time, and the crystal form was measured by powder X-ray diffraction.
The results are shown below.

[Table 3]

| | 5°C | 25°C | 40°C |
|---|---|---|---|
| 0 hr | I + II | I + II | I + II |
| 1 hr | - | II + III | - |
| 2 hr | - | - | III |
| 3 hr | - | III | - |
| 4 hr | II | - | - |
| 24 hr | III | - | - |

The type I crystals and the type II crystals were transformed to the type III crystals.
The type III crystals were more stable than the type I crystals and the type II crystals under nonaqueous conditions.

Test Example 4 Physical stability (3)

The type I crystals and the type III crystals were mixed, suspended in acetonitrile, and stirred at 5, 25, or 40°C.
The mixture was sampled over time, and the crystal form was measured by powder X-ray diffraction.
The results are shown below.

[Table 4]

| | 5°C | 25°C | 40°C |
|---|---|---|---|
| 0 hr | I + III | I + III | I + III |
| 2 hr | - | - | III |
| 3 hr | - | III | - |
| 4 hr | III | - | - |

The type I crystals were transformed to the type III crystals.
The type III crystals were more stable than the type I crystals under nonaqueous conditions.

Test Example 5 Stability (1)

The type I crystals, the type II crystals, and the type III crystals were placed in a light stability tester and irradiated with light (1,200,000 lx·hr) (light source: D65 lamp (FLR20S-D-EDL-D65/M)).
The purity of the crystals of each type was measured by HPLC.
The results are shown below.

HPLC measurement conditions

Measurement wavelength: 254 nm

Column: Ascentis Express C18 (Sigma-Aldrich Japan), particle diameter: 5 μm, inner diameter 4.6 mm × length 150 mm

Column temperature: 40°C

Flow rate: 2.0 mL/min

Mobile phase A: water/acetic acid = 1000/1 solution

Mobile phase B: acetonitrile/acetic acid = 1000/1 solution

Gradient conditions (mobile phase B)

0 - 7.5 min = 5% → 30%

7.5 - 12.5 min = 30% → 90%

12.5 - 17.5 min = 90%

[Table 5]

| | HPLC purity (%) | | |
|---|---|---|---|
| | Type I crystals | Type II crystals | Type III crystals |
| Before start of test | 99.2 | 99.1 | 99.5 |
| 1,200,000 lx·hr | 96.1 | 98.5 | 99.4 |

The purity of the type I crystals was reduced.

The type II crystals and the type III crystals were more stable against light than the type I crystals.

Test Example 6 Stability (2)

The type I crystals, the type II crystals, and the type III crystals were stored under conditions of 60°C and a relative humidity of 75%.

These crystals were sampled over time, and their purity was measured by HPLC. The results are shown below.

HPLC measurement conditions

Measurement wavelength: 254 nm
Column: Ascentis Express C18, particle size: 5 μm, 4.6 mm in inside diameter × 150 mm in length
Column temperature: 40°C
Flow rate: 2.0 mL/min
Mobile phase A: water/acetic acid = 1000/1 solution
Mobile phase B: acetonitrile/acetic acid = 1000/1 solution

Gradient conditions (mobile phase B)

0-7.5 min = 5 → 30%

7.5-12.5 min = 30 → 90%

12.5-17.5 min = 90%

[Table 6]

| | HPLC purity (%) | | |
|---|---|---|---|
| | Type I crystals | Type II crystals | Type III crystals |
| Before start of test | 99.2 | 99.1 | 99.5 |
| 4 weeks | 98.7 | 98.7 | 99.3 |
| 7 weeks | 97.6 | 97.7 | 98.6 |
| 13 weeks | 90.4 | 91.2 | 94.8 |

The HPLC purity of the type I crystals after 13 weeks was approximately 90%.

On the other hand, the HPLC purity of the type II crystals was approximately 91%, and the HPLC purity of the type III crystals was approximately 95%.

The type II crystals and the type III crystals were more stable than the type I crystals.

The type III crystals were most stable.

Test Example 7 Solubility

(1) Solubility of type I crystal and type II crystal

32.4 mL of water for injection was added to 6.00 g of sulfobutyl ether-p-cyclodextrin (CYCLOLAB Cyclodextrin Research & Development Laboratory Ltd.) (hereinafter also referred to as "SBEβCD"), and the solution was then stirred at 25°C for 10 minutes. After the dissolution of the substance had been confirmed by visual observation, the mixed solution was cooled to 10°C. Thereafter, 0.50 g of type I crystal or type II crystal was added to the reaction mixture, and the obtained solution was then heated stepwise, until the crystal was dissolved in the solution.

(2) Solubility of type III crystal

32.5 mL of water for injection was added to 6.00 g of SBEpCD, and the solution was then stirred at 25°C for 10 minutes. After the dissolution of the substance had been confirmed by visual observation, the mixed solution was cooled to 0°C, 5°C or 10°C. Thereafter, 0.48 g of type III crystal was added to the reaction mixture, and the obtained solution was then stirred, until the crystal was dissolved in the solution.

The results are shown below.

[Table 7]

| Temperature (°C) | Type I crystal | Type II crystal | Type III crystal |
|---|---|---|---|
| 0 | NT | NT | + |
| 5 | NT | NT | + |
| 10 | - | - | + |
| 15 | - | - | NT |
| 20 | - | - | NT |
| 25 | + | + | NT |

+: Dissolved
- : Not dissolved
NT: Not tested

The type I crystal and the type II crystal had to be heated up to 25°C, until they were completely dissolved in the solution. On the other hand, the type III crystal was dissolved in the solution even at 0°C.

Examples

Next, the crystals of the present invention will be described with reference to Production Example, Reference Example and Examples. However, the present invention is not intended to be limited by them.

Powder X-ray diffraction was measured using Ultima IV (Rigaku Corp.) under the following conditions.

Measurement conditions

X-ray used: CuKα
Tube voltage: 40 kV
Tube current: 40 mA
Scan axis: 2θ

Water contents were measured using a Karl Fischer moisture meter CA-100 (Mitsubishi Chemical Corp.).

[Formula 3]

Production Example 1

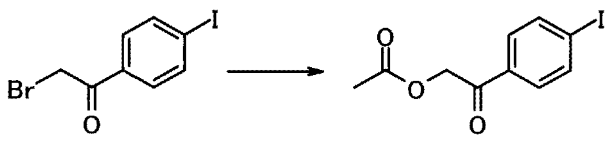

To a mixture of 2-bromo-1-(4-iodophenyl)ethanone (10.00 g), ethanol (39 mL), sodium acetate (3.89 g), and water (19.5 mL), acetic acid (2.03 g) was added in a nitrogen atmosphere, and the resulting mixture was stirred at 70 to 75°C for 3 hours. The reaction mixture was cooled to room temperature. Then, water (19.5 mL) was added thereto, and the mixture was stirred at the same temperature as above for 1 hours. Solid matter was collected by filtration and washed with water (27 mL) twice to obtain 8.92 g of (2-(4-iodophenyl)-2-oxoethyl) acetate as a pale yellow solid.
$^1$H-NMR (600 MHz, $CDCl_3$) $\delta$: 2.23 (3H, s), 5.28 (2H, s), 7.62 (2H, d, J = 9.0 Hz), 7.86 (2H, d, J = 9.0 Hz).

[Formula 4]

Production Example 2

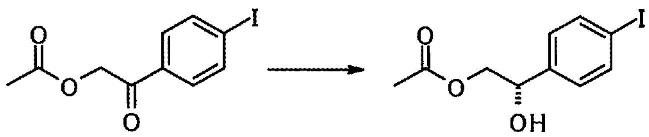

To a mixture of N,N-dimethylformamide (5.0 mL) and N,N-diisopropylethylamine (1.15 mL), formic acid (0.62 mL), (2-(4-iodophenyl)-2-oxoethyl) acetate (1.00 g), and [(R,R)-N-(2-amino-1,2-diphenylethyl)-p-toluenesulfonamido]chloro(p-cymene)ruthenium(II) (10.5 mg) were added in this order at 0 to 10°C in a nitrogen atmosphere, and the resulting mixture was stirred at 20 to 30°C for 6.5 hours. Ethyl acetate (10 mL) and a 20% aqueous sodium chloride solution (10 mL) were added to the reaction mixture. The organic layer was separated and washed with a 20% aqueous sodium chloride solution (5 mL) once, a 5% aqueous sodium bicarbonate solution (5 mL) twice, and a 20% aqueous sodium chloride solution (5 mL) once. Active carbon (0.10 g) was added to the obtained organic layer, and the mixture was stirred at 20 to 30°C for 40 minutes and dried over anhydrous magnesium sulfate. Then, the solvent was distilled off under reduced pressure to obtain 0.99 g of ((2S)-2-hydroxy-2-(4-iodophenyl)ethyl) acetate as a pale yellow oil.
$^1$H-NMR (600 MHz, $CDCl_3$) $\delta$: 2.10 (3H, s), 2.72 (1H, s), 4.10 (1H, dd, J = 11.4, 7.8 Hz), 4.24 (1H, dd, J = 12.0, 3.6 Hz), 4.90 (1H, d, J = 7.8 Hz), 7.14 (2H, d, J = 8.4 Hz), 7.70 (2H, d, J = 8.4 Hz).

[Formula 5]

Production Example 3

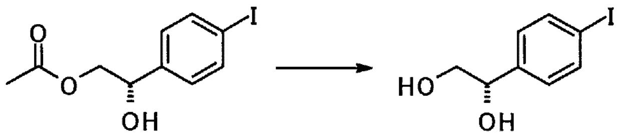

To a mixture of ((2S)-2-hydroxy-2-(4-iodophenyl)ethyl) acetate (0.48 g) and methanol (2.5 mL), potassium carbonate (0.34 g) was added in a nitrogen atmosphere, and the resulting mixture was stirred at room temperature for 2 hours. Methylene chloride (10 mL), water (5 mL), a saturated aqueous solution of sodium chloride (5 mL), and 2-methyl-2-propanol (0.1 mL) were added to the reaction mixture. The organic layer was separated, and the aqueous layer was subjected to extraction with a mixed solution of methylene chloride (10 mL) and 2-methyl-2-propanol (0.1 mL). The organic layer and the extract were combined and dried over anhydrous sodium sulfate. Then, the solvent was distilled off under reduced pressure. Diisopropyl ether was added to the obtained residue, and solid matter was collected by filtration to obtain 0.30 g of (1S)-1-(4-iodophenyl)ethane-1,2-diol as a pale yellow solid.

The obtained solid was used as seed crystals in Production Example 4.

[Formula 57]

Production Example 4

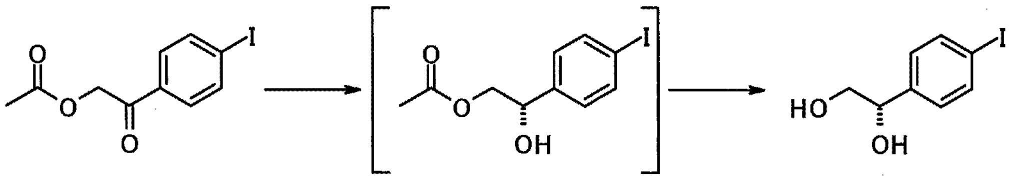

To a mixture of N,N-dimethylformamide (100 mL) and N,N-diisopropylethylamine (17.0 g), formic acid (15.1 g), (2-(4-iodophenyl)-2-oxoethyl) acetate (20.0 g), and [(R,R)-N-(2-amino-1,2-diphenylethyl)-p-toluenesulfonamido]chloro(p-cymene)ruthenium(II) (0.21 g) were added in this order at 0 to 10°C in a nitrogen atmosphere, and the resulting mixture was stirred at 15 to 20°C for 22.5 hours. Ethyl acetate (300 mL) and a 20% aqueous sodium chloride solution (200 mL) were added to the reaction mixture. The organic layer was separated and washed with a 20% aqueous sodium chloride solution (100 mL) once, a 5% aqueous sodium bicarbonate solution (100 mL) twice, and a 20% aqueous sodium chloride solution (100 mL) once. Active carbon (2.00 g) was added to the obtained organic layer, and the mixture was stirred at 20 to 30°C for 1 hour. Insoluble matter was filtered off, and the solvent was distilled off under reduced pressure to obtain ((2S)-2-hydroxy-2-(4-iodophenyl)ethyl) acetate as a pale yellow oil.

To a mixture of the obtained ((2S)-2-hydroxy-2-(4-iodophenyl)ethyl) acetate and methanol (100 mL), potassium carbonate (13.64 g) was added in a nitrogen atmosphere, and the resulting mixture was stirred at 20 to 30°C for 1.5 hours. Insoluble matter was filtered off, and the solvent was distilled off under reduced pressure. Methanol (100 mL) and water (70 mL) were added to the obtained residue. After confirmation of dissolution, the seed crystals (0.05 g) were added to the solution, and the mixture was stirred at 20 to 30°C for 2 hours. Water (10 mL) and 6 mol/L hydrochloric acid (11.3 mL) were added to the reaction mixture for pH adjustment to 7 to 8, and the mixture was then stirred at 20 to 30°C for 2 hours and left standing overnight. Water (100 mL) was added to the reaction mixture over 1 hour, and the mixture was stirred at the same temperature as above for 2.5 hours. Water (100 mL) was added to the reaction mixture over 2 hours, and the mixture was stirred at the same temperature as above for 1.5 hours. Water (220 mL) was added to the reaction mixture over 1.5 hours, and the mixture was stirred at the same temperature as above for 1 hour. The reaction mixture was cooled to 0 to 10°C and then stirred at the same temperature as above for 1 hour. Solid matter was collected by filtration and washed with a 10% aqueous methanol solution (40 mL) twice to obtain 15.4 g of (1S)-1-(4-iodophenyl)ethane-1,2-diol as a pale yellow solid.

Optical purity: 96.2% ee
HPLC measurement conditions

Measurement wavelength: 230 nm
Column: CHIRALPAK ID (Daicel Corporation), particle diameter: 5 μm, inner diameter 4.6 mm × length 250 mm
Column temperature: 40°C
Flow rate: 1.0 mL/min
Mobile phase: hexane/ethanol = 970/30

$^1$H-NMR (600 MHz, DMSO-$d_6$) δ: 3.35-3.44 (2H, m), 4.48 (1H, dd, J = 11.4, 5.4 Hz), 4.73 (1H, t, J = 6.6 Hz), 5.31 (1H, d, J = 4.2 Hz), 7.15 (2H, d, J = 7.8 Hz), 7.66 (2H, d, J = 8.4 Hz).

[Formula 7]

Production Example 5

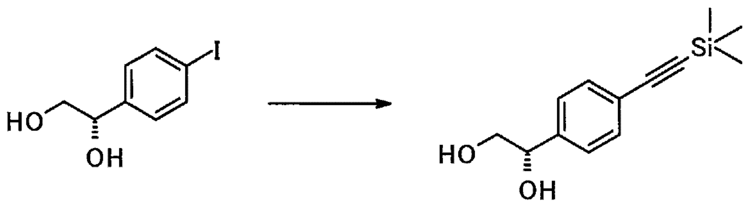

To a mixture of (1S)-1-(4-iodophenyl)ethane-1,2-diol (10.0 g), bis(triphenylphosphine)palladium(II) dichloride (0.13 g), copper(I) iodide (0.11 g), triethylamine (9.96 g), and tetrahydrofuran (50 mL), a mixture of trimethylsilylacetylene (4.09 g) and tetrahydrofuran (20 mL) was added over 1 hour in a nitrogen atmosphere, and the resulting mixture was stirred at room temperature for 1 hour. Ethyl acetate (100 mL) and a 5% aqueous ammonium sulfate solution (50 mL) were added to the reaction mixture. The organic layer was separated and washed with a 40% aqueous ammonium sulfate solution (50 mL), a 10% aqueous N-acetylcysteine solution (50 mL), a 5% aqueous sodium bicarbonate solution (50 mL), and a mixed solution of a 5% aqueous sodium bicarbonate solution and a 20% aqueous sodium chloride solution (50 mL) in this order. Anhydrous sodium sulfate (20 g) and SH SILICA (1.0 g) were added to the obtained organic layer, and the mixture was stirred at room temperature for 30 minutes. Active carbon (0.5 g) was added to the obtained mixture, and the resulting mixture was stirred at room temperature. Insoluble matter was filtered off, and the solvent was distilled off under reduced pressure. Heptane and isopropyl acetate were added to the obtained residue, and solid matter was collected by filtration and washed with a mixed solution of heptane and isopropyl acetate to obtain 7.10 g of (1S)-1-(4-((trimethylsilyl)ethynyl)phenyl)ethane-1,2-diol as a white solid.
$^1$H-NMR (600 MHz, $CDCl_3$) δ: 0.25 (9H, s), 2.42-2.49 (1H, m), 2.92-3.00 (1H, m), 3.54-3.63 (1H, m), 3.67-3.75 (1H, m), 4.74-4.81 (1H, m), 7.28 (2H, d, J = 8.4 Hz), 7.45 (2H, d, J = 8.4 Hz).

[Formula 8]

Production Example 6

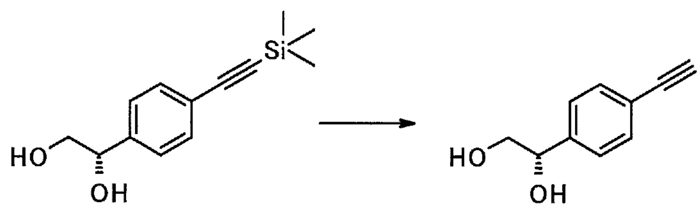

To a mixture of (1S)-1-(4-((trimethylsilyl)ethynyl)phenyl)ethane-1,2-diol (0.36 g) and methanol (3 mL), potassium carbonate (0.24 g) was added in a nitrogen atmosphere, and the resulting mixture was stirred at room temperature for 1.5 hours. Water (3 mL) was added to the reaction mixture, and the mixture was stirred at room temperature for 1 hour. Insoluble matter was filtered off, and methylene chloride was added to the filtrate. The organic layer was separated, and the aqueous layer was subjected to extraction with methylene chloride three times. The organic layer and the extracts were combined and dried over anhydrous sodium sulfate. Then, the solvent was distilled off under reduced pressure. Hexane and isopropyl acetate were added to the obtained residue, and solid matter was collected by filtration to obtain 0.14 g of (1S)-1-(4-ethynylphenyl)ethane-1,2-diol as a pale yellow solid.

The obtained solid was used as seed crystals in Production Example 7.

[Formula 9]

Production Example 7

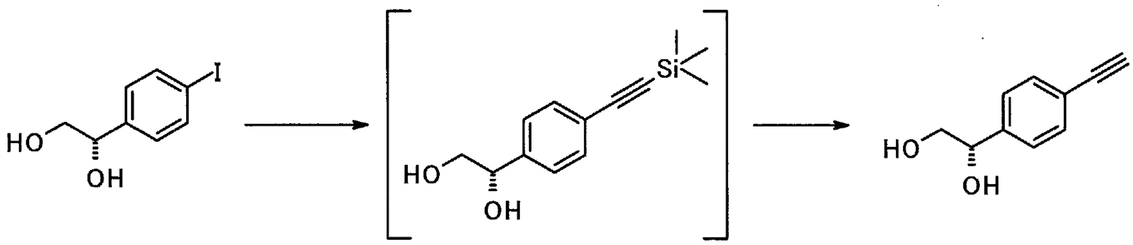

(1S)-1-(4-((Trimethylsilyl)ethynyl)phenyl)ethane-1,2-diol was obtained as a pale yellow solid in the same way as in Example 4 using (1S)-1-(4-iodophenyl)ethane-1,2-diol (5.00 g).

To a mixture of the obtained (1S)-1-(4-((trimethylsilyl)ethynyl)phenyl)ethane-1,2-diol and methanol (25 mL), potassium carbonate (3.93 g) was added in a nitrogen atmosphere, and the resulting mixture was stirred at 20 to 30°C for 1.5 hours. Active carbon (0.50 g) was added to the reaction mixture, and the mixture was stirred at 20 to 30°C for 1 hour. Insoluble matter was filtered off, and the solvent was distilled off under reduced pressure. Methanol (6.2 mL) and water (6.2 mL) were added to the obtained residue at 20 to 30°C. After confirmation of dissolution, 6 mol/L hydrochloric acid was added to the solution for pH adjustment to 6 to 7. Water (7.8 mL) and a 25% aqueous sodium chloride solution (6.2 mL) were added to the obtained mixture at the same temperature as above, then the seed crystals were added thereto, and the resulting mixture was then stirred at the same temperature as above for 1.5 hours. A 25% aqueous sodium chloride solution (6.2 mL) was added to the obtained mixture, and the resulting mixture was then stirred at 20 to 30°C for 1 hour. A 25% aqueous sodium chloride solution (6.2 mL) was added to the obtained mixture, and the resulting mixture was stirred at the same temperature as above for 4 hours and left standing overnight. The obtained mixture was stirred at 0 to 10°C for 1.5 hours. Solid matter was collected by filtration and washed with cold water (6.2 mL) twice to obtain 2.63 g of (1S)-1-(4-ethynylphenyl)ethane-1,2-diol as a pale yellow solid.

Optical purity: >99.9% ee
HPLC measurement conditions

Measurement wavelength: 230 nm
Column: CHIRALPAK ID (Daicel Corporation), particle diameter: 5 $\mu$m, inner diameter 4.6 mm $\times$ length 250 mm
Column temperature: 40°C
Flow rate: 1.0 mL/min
Mobile phase: hexane/ethanol = 970/30

To a mixture of the obtained (1S)-1-(4-ethynylphenyl)ethane-1,2-diol (2.00 g) and ethyl acetate (40 mL), active carbon (0.20 g) was added in a nitrogen atmosphere, and the resulting mixture was stirred at 20 to 30°C for 1 hour. Insoluble matter was filtered off, and the solvent was distilled off under reduced pressure. Ethyl acetate (10 mL) was added to the obtained residue. After confirmation of dissolution, heptane (10 mL) was added to the solution, then the seed crystals were added thereto, and the mixture was then stirred at 20 to 30°C for 3 hours and left standing overnight. Heptane (15 mL) was added to the obtained mixture over 1 hour, and the resulting mixture was stirred at 20 to 30°C for 1 hour. Heptane (15 mL) was added to the obtained mixture over 1 hour, and the resulting mixture was stirred at the same temperature as above for 1 hour. The obtained mixture was cooled to 0 to 10°C and then stirred at the same temperature as above for 2 hours. Solid matter was collected by filtration and washed with heptane to obtain 1.67 g of (1S)-1-(4-ethynylphenyl)ethane-1,2-diol as a white solid.
$^1$H-NMR (400 MHz, DMSO-$d_6$) $\delta$: 3.37-3.49 (2H, m), 4.11 (1H, s), 4.55 (1H, dd, J = 10.4, 5.6 Hz), 4.74 (1H, t, J = 5.6 Hz), 5.33 (1H, d, J = 4.4 Hz), 7.35 (2H, d, J = 8.4 Hz), 7.42 (2H, d, J = 8.0 Hz).

[Formula 10]

Production Example 8

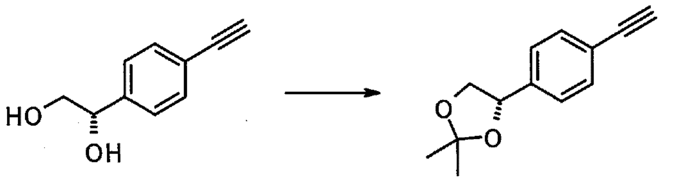

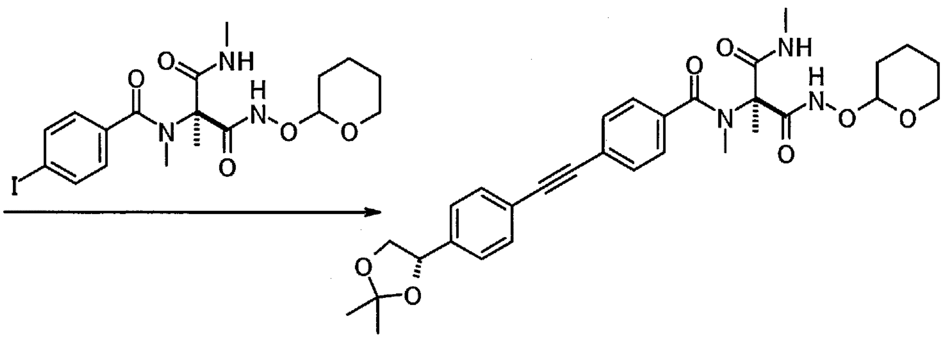

(1) To a mixture of (1S)-1-(4-ethynylphenyl)ethane-1,2-diol (6.96 g) and N-methylpyrrolidone (21 mL), 2,2-dimethoxypropane (13.4 g) and methanesulfonic acid (279 μL) were added in a nitrogen atmosphere, and the resulting mixture was stirred at 20 to 30°C for 5.5 hours to obtain a solution.
(2) A mixture of (2S)-2-((4-iodobenzoyl)(methyl)amino)-N,2-dimethyl-N'-(tetrahydro-2H-pyran-2-yloxy)malo namide (20.0 g), bis(triphenylphosphine)palladium(II) dichloride (0.22 g), copper(I) iodide (0.12 g), triethylamine (10.3 g), and N-methylpyrrolidone (40 mL) was stirred at 30 to 40°C for 1 hour in a nitrogen atmosphere. The solution obtained in (1) was added to the reaction mixture at the same temperature as above over 2.5 hours, and the mixture was stirred at the same temperature as above for 3.5 hours to obtain a reaction mixture.
(3) The same operation as in (1) and (2) was carried out three times. All of the obtained reaction mixtures were combined. 2-Methyltetrahydrofuran (200 mL) and a 5% aqueous ammonium sulfate solution (300 mL) were added to this reaction mixture, and 6 mol/L hydrochloric acid (31 mL) was then added thereto for pH adjustment to 6 to 8. The organic layer was separated.
(4) The same operation as in (1) and (2) was carried out. 2-Methyltetrahydrofuran (200 mL) was added to the obtained reaction mixture, and the mixture was cooled to 0 to 10°C. A 5% aqueous ammonium sulfate solution (100 mL) was added to the obtained mixture, and 6 mol/ hydrochloric acid (17.7 mL) was then added thereto for pH adjustment to 6 to 7. The organic layer was separated.
(5) The same operation as in (4) was carried out.
(6) The organic layers obtained in (3), (4), and (5) were combined and washed with a 40% aqueous ammonium sulfate solution (500 mL) once, a 10% aqueous N-acetylcysteine solution (450 mL) once, a 2.5% aqueous sodium bicarbonate solution (500 mL) twice, and a 5% aqueous sodium chloride solution (500 mL) once, and the solvent was distilled off under reduced pressure. Isopropyl acetate (1200 mL) was added to the obtained residue, and the solvent was distilled off under reduced pressure. Isopropyl acetate was added to the obtained residue, and the mixture was heated to 50°C, then cooled to 25°C over 5 hours, and left standing overnight. Solid matter was collected by filtration and washed with cyclopentyl methyl ether twice to obtain 64.8 g of (2S)-2-((4-((4-((4S)-2,2-dimethyl-1,3-dioxolan-4-yl)phenyl)ethynyl)benzoyl)(methyl)amino)-N,2-dimethyl-N'-(tetrahydro-2H-pyran-2-yloxy)malonamide as a pale yellow solid.

The obtained solid was used as seed crystals in Production Example 9.

[Formula 11]

Production Example 9

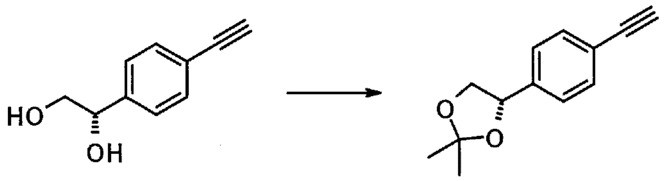

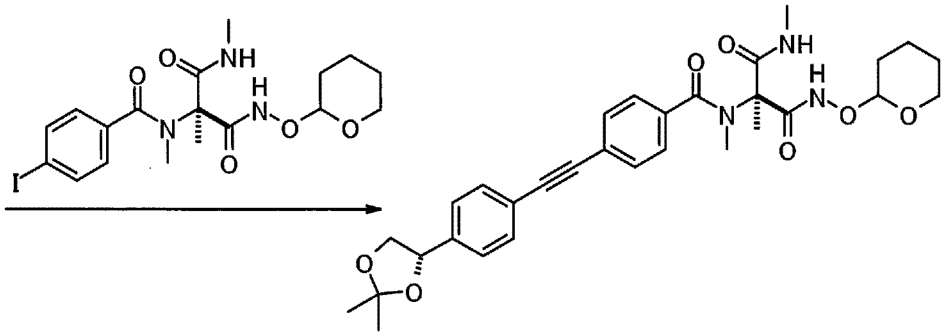

(1) To a mixture of (1S)-1-(4-ethynylphenyl)ethane-1,2-diol (6.96 g) and N-methylpyrrolidone (21 mL), 2,2-dimethoxypropane (13.4 g) and methanesulfonic acid (279 μL) were added in a nitrogen atmosphere, and the resulting mixture was stirred at 20 to 30°C for 5 hours to obtain a solution.

(2) A mixture of (2S)-2-((4-iodobenzoyl)(methyl)amino)-N,2-dimethyl-N'-(tetrahydro-2H-pyran-2-yloxy)malo namide (20.0 g), bis(triphenylphosphine)palladium(II) dichloride (0.22 g), copper(I) iodide (0.12 g), triethylamine (14.5 g), and N-methylpyrrolidone (40 mL) was stirred at 30 to 40°C for 1 hour in a nitrogen atmosphere. The solution obtained in (1) was added to the reaction mixture at the same temperature as above over 3 hours, and the mixture was stirred at the same temperature as above for 2 hours. Ethyl acetate (200 mL) was added to the reaction mixture, and the mixture was cooled to 0 to 10°C. A 5% aqueous ammonium sulfate solution (100 mL) was added thereto, and 6 mol/L hydrochloric acid (14.5 mL) and a 5% aqueous sodium bicarbonate solution (4.5 mL) were then added thereto for pH adjustment to 6 to 7. The organic layer was separated and washed with a 40% aqueous ammonium sulfate solution (100 mL) once, a 10% aqueous N-acetylcysteine solution (100 mL) once, a 5% aqueous sodium bicarbonate solution (100 mL) twice, and water (100 mL) once, and the solvent was distilled off under reduced pressure. Ethyl acetate (160 mL) was added to the obtained residue. After confirmation of dissolution, the solution was heated to 30 to 35°C. Heptane (150 mL) was added to the obtained mixture, then the seed crystals (100 mg) were added thereto, and the resulting mixture was stirred at the same temperature as above for 1 hour. Heptane (50 mL) was added to the obtained mixture, and the resulting mixture was stirred at 30 to 35°C for 1 hour. Heptane (200 mL) was added to the obtained mixture over 1 hour, and the resulting mixture was stirred at the same temperature as above for 1 hour. The obtained mixture was cooled to 20 to 30°C, stirred at the same temperature as above for 30 minutes, and then left standing overnight. The obtained mixture was cooled to 0 to 10°C and stirred at the same temperature as above for 1 hour. Then, solid matter was collected by filtration and washed with a mixed solvent of ethyl acetate and heptane (ethyl acetate:heptane = 1:10) (40 mL) twice to obtain 20.5 g of (2S)-2-((4-((4-((4S)-2,2-dimethyl-1,3-dioxolan-4-yl)phenyl)ethynyl)benzoyl)(methyl)amino)-N,2-dimethyl-N'-(tetrahydro-2H-pyran-2-yloxy)malonamide as a pale yellow solid.

$^1$H-NMR (600 MHz, $CDCl_3$) δ: 1.50 (3H, s), 1.52-1.74 (3H, m), 1.56 (3H, s), 1.75-1.90 (3H, m), [1.81], 1.82 (3H, s), [2.84], 2.85 (3H, d, J = 4.8 Hz), [3.17], 3.20 (3H, s), [3.53-3.60], 3.62-3.68 (1H, m), 3.70 (1H, t, J = 7.8 Hz), [3.83-3.91], 3.98-4.06 (1H, m), 4.33 (1H, dd, J = 8.4, 6.0 Hz), [4.92-4.98], 4.98-5.03 (1H, m), 5.09 (1H, t, J = 6.0 Hz), [6.98-7.05], 7.61-7.67 (1H, m), 7.37 (2H, d, J = 8.4 Hz), 7.47-7.55 (4H, m), 7.58 (2H, d, J = 8.4 Hz), [10.14], 10.54 (1H, s).

[Formula 12]

Production Example 10

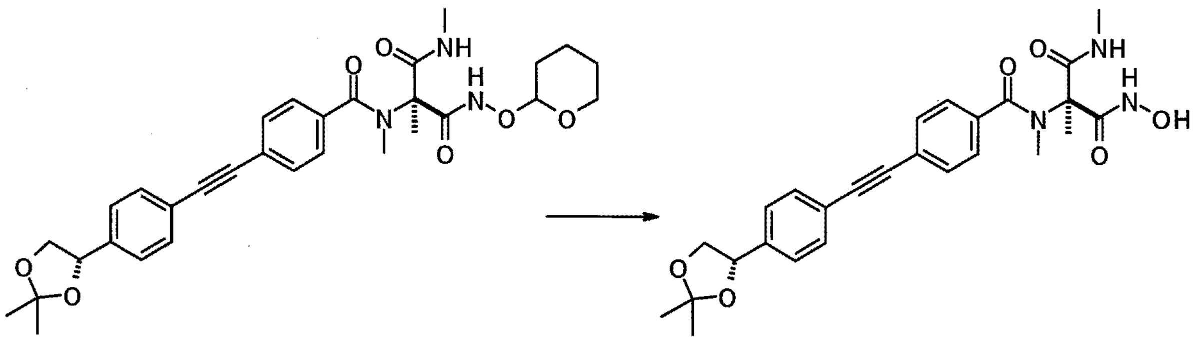

To a mixture of (2S)-2-((4-((4-((4S)-2,2-dimethyl-1,3-dioxolan-4-yl)phenyl)ethynyl)benzoyl)(methyl)amino)-N,2-dimethyl-N'-(tetrahydro-2H-pyran-2-yloxy)malonamide (3.46 g), acetone (3 mL), and 2-propanol (21 mL), methanesulfonic acid (121 μL) was added at 0 to 10°C in a nitrogen atmosphere, and the resulting mixture was stirred at the same temperature as above for 48 hours. 2-Methyltetrahydrofuran (60 mL), a 20% aqueous sodium chloride solution (60 mL), and a 5% aqueous sodium bicarbonate solution (6 mL) were added to the reaction mixture. The organic layer was separated and washed with a 20% aqueous sodium chloride solution (60 mL). Then, active carbon (0.30 g) was added thereto, and the mixture was stirred at 20 to 30°C for 2.5 hours. Insoluble matter was filtered off, and the solvent was distilled off under reduced pressure. Acetonitrile (12 mL) and water (3 mL) were added to the obtained residue, and the mixture was heated to 35 to 40°C. After confirmation of dissolution, water (12.7 mL) was added to the solution, and the mixture was stirred at the same temperature as above for 1 hour. The obtained mixture was cooled to 20 to 30°C, then stirred at the same temperature as above for 1 hour, and left standing overnight. Water (47.9 mL) was added to the obtained mixture over 1 hour, and the resulting mixture was stirred at 20 to 30°C for 1 hour, then cooled to 0 to 10°C, and stirred at the same temperature as above for 3.5 hours. Solid matter was collected by filtration and washed with a 10% aqueous acetonitrile solution (6 mL) twice to obtain 2.65 g of (2S)-2-((4-((4-((4S)-2,2-dimethyl-1,3-dioxolan-4-yl)phenyl)ethynyl)benzoyl)(methyl)amino)-N-hydroxy-N',2-dimethylmalonamide as a white solid.
$^1$H-NMR (600 MHz, DMSO-$d_6$) δ: 1.41 (3H, s), 1.47 (3H, s), 1.62 (3H, s), 2.65 (3H, d, J = 4.8 Hz), 3.01 (3H, s), 3.59 (1H, t, J = 8.4 Hz), 4.33 (1H, dd, J = 8.4, 7.2 Hz), 5.11 (1H, t, J = 6.6 Hz), 7.44 (2H, d, J = 8.4 Hz), 7.59 (4H, d, J = 8.4 Hz), 7.65 (2H, d, J = 8.4 Hz), 8.47-8.57 (1H, m), 8.98 (1H, s), 10.97 (1H, s).

Reference Example 1 Type I crystals

The type I crystals were obtained by the method described in Patent Literature 1.
The infrared absorption spectrum (ATR method) is shown in Figure 1.
The powder X-ray diffraction pattern is shown in Figure 2 and Table8.
Water content: 4.1%

[Table 8]

| 2θ (°) | d value (Å) | Relative intensity (%) |
|---|---|---|
| 3.5 | 25.2 | 62 |
| 12.7 | 7.0 | 28 |
| 16.2 | 5.5 | 51 |
| 16.5 | 5.4 | 100 |
| 22.4 | 4.0 | 68 |
| 22.7 | 3.9 | 57 |
| 23.0 | 3.9 | 36 |
| 24.2 | 3.7 | 29 |

Example 1 Type II crystals

A suspension of 0.20 g of the type I crystals in 2 mL of 50% aqueous acetonitrile was stirred at 20 to 30°C for 21 hours. Solid matter was collected by filtration to obtain 0.13 g of type II crystals as a white solid.
The infrared absorption spectrum (ATR method) is shown in Figure 3.
The powder X-ray diffraction pattern is shown in Figure 4 and Table 9.

Water content: 3.9%

IR (ATR): 1475, 1606, 1683, 3134, and 3365 $cm^{-1}$

[Table 9]

| 2θ (°) | d value (Å) | Relative intensity (%) |
|---|---|---|
| 3.8 | 23.1 | 44 |
| 7.7 | 11.5 | 64 |
| 10.8 | 8.2 | 21 |
| 12.0 | 7.4 | 32 |
| 14.4 | 6.1 | 22 |
| 15.6 | 5.7 | 38 |
| 16.3 | 5.4 | 93 |
| 17.0 | 5.2 | 100 |
| 21.8 | 4.1 | 88 |
| 22.3 | 4.0 | 32 |
| 22.8 | 3.9 | 42 |

[Formula 13]

Example 2 Type II crystals

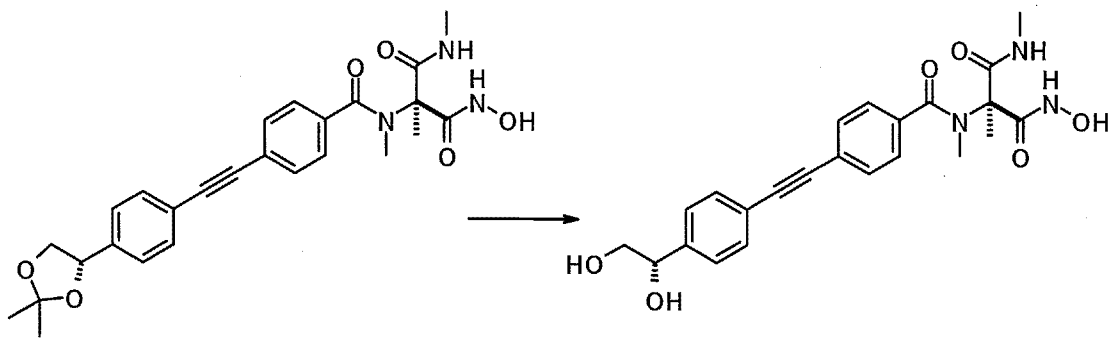

To a mixture of (2S)-2-((4-((4-((4S)-2,2-dimethyl-1,3-dioxolan-4-yl)phenyl)ethynyl)benzoyl)(methyl)amino)-N-hydroxy-N',2-dimethylmalonamide (180 g) and 2-propanol (900 mL), 1 mol/L hydrochloric acid (360 mL) was added at 18 to 20°C, and the resulting mixture was stirred at 20 to 26°C for 10 hours and then left standing overnight. Water (540 mL) was added to the reaction mixture at 24 to 25°C, and the mixture was stirred at 25°C for 45 minutes. Solid matter was collected by filtration and washed with a 50% aqueous 2-propanol solution (180 mL), water (540 mL), and an 80% aqueous 2-propanol solution (540 mL) in this order to obtain 114 g of (2S)-2-((4-((4-((1S)-1,2-dihydroxyethyl)phenyl)ethynyl)benzoyl)(methyl)amino)-N-hydroxy-N',2-dimethylmalonamide hydrate as a white solid.

The obtained solid was used as seed crystals in Example 3.

[Formula 14]

Example 3 Type II crystals

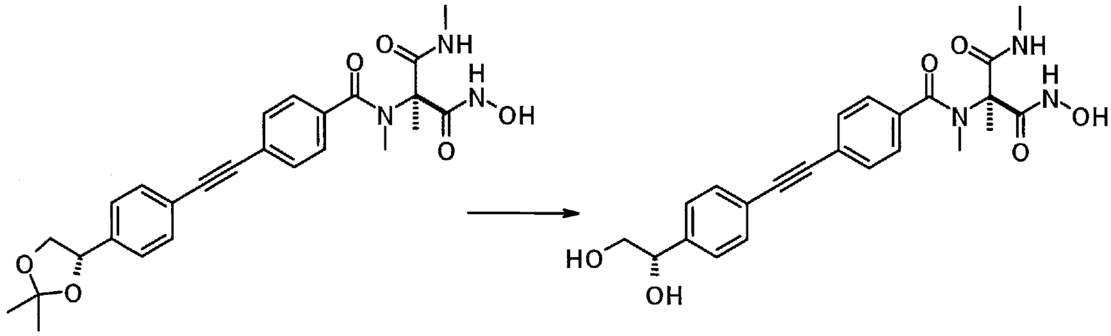

To a mixture of (2S)-2-((4-((4-((4S)-2,2-dimethyl-1,3-dioxolan-4-yl)phenyl)ethynyl)benzoyl)(methyl)amino)-N-hydroxy-N',2-dimethylmalonamide (2.00 g) and acetonitrile (20 mL), 1 mol/L hydrochloric acid (4 mL) was added at 5 to 10°C, and the resulting mixture was stirred at the same temperature as above for 18.5 hours. A mixture of sodium bicarbonate (0.34 g) and water (8.1 mL) was added to the reaction mixture, and the resulting mixture was heated to 50 to 60°C. After confirmation of dissolution, the pH of the solution was adjusted to 5 to 6 using a 5% aqueous sodium bicarbonate solution and 1 mol/L hydrochloric acid. The obtained mixture was cooled to 35 to 40°C. The seed crystals (10 mg) were added thereto, and the mixture was stirred at the same temperature as above for 1 hour. The obtained mixture was cooled to 30 to 35°C. Water (47 mL) was added thereto over 2 hours, and the mixture was stirred at the same temperature as above for 1 hour. The obtained mixture was cooled to 22.5 to 27.5°C, stirred at the same temperature as above for 1 hour, and then left standing overnight. The obtained mixture was cooled to 12.5 to 17.5°C and stirred at the same temperature as above for 1 hour. The obtained mixture was cooled to 0 to 5°C and then stirred at the same temperature as above for 3 hours. Solid matter was collected by filtration and washed with a 10% aqueous acetonitrile solution (2 mL) twice to obtain 1.62 g of (2S)-2-((4-((4-((1S)-1,2-dihydroxyethyl)phenyl)ethynyl)benzoyl)(methyl)amino)-N-hydroxy-N',2-dimethylmalonamide hydrate as a white solid.

The attenuated total reflection infrared spectroscopy (ATR method) is shown in Figure 5.

The powder X-ray diffraction pattern is shown in Figure 6 and Table 10.

Water content: 4.1%
IR (ATR): 1474, 1605, 1682, 3132, 3363cm$^{-1}$
Optical purity: >99%ee (>99%de)
HPLC measurement conditions

Measurement wavelength : 290 nm
Column: CHIRALCEL OZ-H (Daicel Corporation), particle diameter: 5 $\mu$m, inner diameter 4.6 mm $\times$ length 250 mm
Column temperature: 40°C
Flow rate: 0.7 mL/min
Mobile phase: hexane/ethanol/acetic acid = 650/350/5

$^1$H-NMR (600MHz, DMSO-$d_6$) $\delta$ value: 1.62 (3H, s), 2.65 (3H, d, J = 3.6Hz), 3.01 (3H, s), 3.40-3.51 (2H, m), 4.53-4.62 (1H, m), 4.77 (1H, t, J = 5.4Hz), 5.36 (1H, d, J = 4.2Hz), 7.41 (2H, d, J = 7.8Hz), 7.53 (2H, d, J = 7.8Hz), 7.58 (2H, d, J = 7.8Hz), 7.64 (2H, d, J = 8.4Hz), 8.52 (1H, d, J = 4.2Hz), 8.98 (1H, s), 10.97 (1H, s).

[Table 10]

| 2θ (°) | d value (Å) | Relative intensity (%) |
|---|---|---|
| 3.8 | 23.1 | 74 |
| 7.7 | 11.5 | 100 |
| 10.8 | 8.2 | 6 |
| 12.0 | 7.3 | 12 |
| 14.4 | 6.1 | 11 |
| 15.4 | 5.7 | 25 |
| 16.3 | 5.4 | 24 |
| 17.0 | 5.2 | 33 |
| 21.8 | 4.1 | 23 |

(continued)

| 2θ (°) | d value (Å) | Relative intensity (%) |
|---|---|---|
| 22.3 | 4.0 | 10 |
| 22.8 | 3.9 | 13 |

Example 4 Type III crystals

A suspension of 150 g of the type II crystals in 450 mL of methanol was stirred at 20 to 25°C for 3 hours and 20 minutes and then stirred for 15 minutes under ice cooling. Solid matter was collected by filtration to obtain 108 g of type III crystals as a white solid.

The infrared absorption spectrum (ATR method) is shown in Figure 7.

The powder X-ray diffraction pattern is shown in Figure 8 and Table 11.

Water content: 0.2%
IR (ATR): 1484, 1608, 1688, 3288, and 3475 $cm^{-1}$

[Table 11]

| 2θ (°) | d value (Å) | Relative intensity (%) |
|---|---|---|
| 8.2 | 10.7 | 33 |
| 12.4 | 7.2 | 38 |
| 13.3 | 6.6 | 33 |
| 15.2 | 5.8 | 38 |
| 15.8 | 5.6 | 49 |
| 16.2 | 5.5 | 85 |
| 16.6 | 5.3 | 46 |
| 18.4 | 4.8 | 62 |
| 19.0 | 4.7 | 98 |
| 19.4 | 4.6 | 44 |
| 20.2 | 4.4 | 100 |
| 20.9 | 4.2 | 40 |
| 21.2 | 4.2 | 74 |
| 21.5 | 4.1 | 57 |
| 22.8 | 3.9 | 65 |
| 23.4 | 3.8 | 36 |

Example 5 Type III crystals

A suspension of 16.5 g of the type II crystals in 49.5 mL of methanol was stirred at 20 to 30°C for 3 hours. Solid matter was collected by filtration, and washed twice with 33mL of methanol to obtain 12.0 g of type III crystals as a white solid.

The infrared absorption spectrum (ATR method) is shown in Figure 9.

The powder X-ray diffraction pattern is shown in Figure 10 and Table 12.

The obtained solid was used as seed crystals in Example 11.

Water content: 0.3%
IR(ATR):1481,1607,1688,3286,3475$cm^{-1}$
$^1$H-NMR(400MHz,DMSO-$d_6$)δ value: 1.63(3H,s), 2.65(3H,d,J=4.8Hz), 3.02(3H,s), 3.40-3.51(2H,m), 4.53-4.62(1H,m), 4.77(1H,t,J=5.8Hz), 5.37(1H,d,J=4.4Hz), 7.41(2H,d,J=8.0Hz), 7.53(2H,d,J=8.4Hz), 7.59(2H,d,J=8.0Hz), 7.64(2H,d,J=8.4Hz), 8.46-8.58(1H,m), 8.99(1H,d,J=1.2Hz), 10.98(1H,s).

[Table 12]

| 2θ (°) | d value (Å) | Relative intensity (%) |
|---|---|---|
| 8.3 | 10.7 | 33 |
| 12.4 | 7.1 | 40 |
| 13.4 | 6.6 | 31 |
| 15.2 | 5.8 | 40 |
| 15.8 | 5.6 | 52 |
| 16.2 | 5.5 | 90 |
| 16.6 | 5.3 | 47 |
| 18.5 | 4.8 | 64 |
| 19.1 | 4.7 | 100 |
| 19.4 | 4.6 | 42 |
| 20.3 | 4.4 | 100 |
| 20.9 | 4.2 | 38 |
| 21.2 | 4.2 | 77 |
| 21.5 | 4.1 | 60 |
| 22.9 | 3.9 | 67 |
| 23.4 | 3.8 | 39 |

Example 6 Type III crystal

Type III crystal was obtained in the same manner as that of Example 4, with the exception that ethanol was used instead of methanol.

Example 7 Type III crystal

Type III crystal was obtained in the same manner as that of Example 4, with the exception that ethyl acetate was used instead of methanol.

Example 8 Type III crystal

Type III crystal was obtained in the same manner as that of Example 4, with the exception that tetrahydrofuran was used instead of methanol.

Example 9 Type III crystal

Type III crystal was obtained in the same manner as that of Example 4, with the exception that acetonitrile was used instead of methanol.

Example 10 Type III crystal

Type III crystal was obtained in the same manner as that of Example 4, with the exception that type I crystal was used instead of type II crystal.

Example 11 Type III crystal

To a mixed solution of methanol (2 mL) and dimethyl sulfoxide (0.5 mL), type II crystal (1.00 g) was added at a temperature of 0°C to 10°C under a nitrogen atmosphere, and the obtained mixture was then stirred at the same temperature as mentioned above for 4 minutes. After conformation of the dissolution of the crystal, the temperature was increased to a temperature of 20°C to 25°C, and methanol (2 mL) and ethyl acetate (2 mL) were then added to the reaction mixture. Thereafter, a seed crystal of the type III crystal was added to the mixture, and the thus obtained mixture was then stirred at the same temperature as mentioned above for 2 hours. Thereafter, 8 mL of ethyl acetate was added to the reaction mixture at a temperature of 20°C to 25°C over 30 minutes, and was then stirred at the same temperature as mentioned above for 1 hour. Subsequently, heptane (10 mL) was added to the reaction mixture at a temperature of 20°C to 25°C over 30 minutes, and was then stirred at the same temperature as mentioned above for 1 hour. After that,

the reaction mixture was cooled to a temperature of 0°C to 10°C, and was then stirred at the same temperature as mentioned above for 1 hour. A solid was collected by filtration, and was then washed twice with ethyl acetate (2 mL) to obtain type III crystal (0.90 g) as a white solid.

The attenuated total reflection infrared spectroscopy (ATR method) and the powder X-ray diffraction pattern were matched with those of Example 5.

Water content: 0.1%
Optical purity: >99%ee (>99%de)
HPLC measurement conditions

Measurement wavelength : 290 nm
Column: CHIRALCEL OZ-H (Daicel Corporation), particle diameter: 5 μm, inner diameter 4.6 mm × length 250 mm
Column temperature: 40°C
Flow rate: 0.7 mL/min
Mobile phase: hexane/ethanol/acetic acid = 650/350/5

Industrial Applicability

The crystals of the present invention are excellent in stability, easily handleable, and useful as a bulk pharmaceutical.

**Claims**

1. A crystal of a hydrate of (2S)-2-((4-((4-((1S)-1,2-dihydroxyethyl)phenyl)ethynyl)benzoyl)(methyl)amino)-N-hydroxy-N',2-dimethylmalonamide having diffraction peaks at diffraction angles (2θ) of 3.8 ± 0.2°, 7.7 ± 0.2°, 10.8 ± 0.2°, 12.0 ± 0.2°, and 14.4 ± 0.2° in powder X-ray diffraction.

2. A crystal of a hydrate of (2S)-2-((4-((4-((1S)-1,2-dihydroxyethyl)phenyl)ethynyl)benzoyl)(methyl)amino)-N-hydroxy-N',2-dimethylmalonamide having diffraction peaks at diffraction angles (2θ) of 3.8 ± 0.2°, 7.7 ± 0.2°, 10.8 ± 0.2°, 12.0 ± 0.2°, 14.4 ± 0.2°, 16.3 ± 0.2°, 17.0 ± 0.2°, and 21.8 ± 0.2° in powder X-ray diffraction.

3. A crystal of (2S)-2-((4-((4-((1S)-1,2-dihydroxyethyl)phenyl)ethynyl)benzoyl)(methyl)amino)-N-hydroxy-N',2-dimethylmalonamide having diffraction peaks at diffraction angles (2θ) of 8.2 ± 0.2°, 12.4 ± 0.2°, 13.3 ± 0.2°, 15.2 ± 0.2°, and 16.2 ± 0.2° in powder X-ray diffraction.

4. A crystal of (2S)-2-((4-((4-((1S)-1,2-dihydroxyethyl)phenyl)ethynyl)benzoyl)(methyl)amino)-N-hydroxy-N',2-dimethylmalonamide having diffraction peaks at diffraction angles (2θ) of 8.2 ± 0.2°, 12.4 ± 0.2°, 13.3 ± 0.2°, 15.2 ± 0.2°, 16.2 ± 0.2°, 19.0 ± 0.2°, 20.2 ± 0.2°, and 22.8 ± 0.2° in powder X-ray diffraction.

5. A pharmaceutical composition which comprises the crystal according to any one of claims 1 to 4.

6. A method for producing the crystal according to claim 1 or 2, comprising stirring a mixture containing (1) (2S)-2-((4-((4-((1S)-1,2-dihydroxyethyl)phenyl)ethynyl)benzoyl)(methyl)amino)-N-hydroxy-N',2-dimethylmalonamide, (2) water, and (3) an organic solvent, wherein the organic solvent is one or two or more selected from alcohols, ethers, ketones, and nitriles.

7. The production method according to claim 6, wherein the alcohols are methanol, ethanol and 2-propanol, the ether is tetrahydrofuran, the ketone is acetone, and the nitrile is acetonitrile.

8. The production method according to claim 6 or 7, wherein (2S)-2-((4-((4-((1S)-1,2-dihydroxyethyl)phenyl)ethynyl)benzoyl)(methyl)amino)-N-hydroxy-N',2-dimethylmalonamide is a crystal of a hydrate of (2S)-2-((4-((4-((1S)-1,2-dihydroxyethyl)phenyl)ethynyl)benzoyl)(methyl)amino)-N-hydroxy-N',2-dimethylmalonamide having diffraction peaks at diffraction angles (2θ) of 3.5 ± 0.2°, 16.2 ± 0.2°, 16.5 ± 0.2°, 22.4 ± 0.2°, and 22.7 ± 0.2° in powder X-ray diffraction.

9. A method for producing the crystal according to claim 3 or 4, comprising stirring a mixture containing (1) (2S)-2-((4-((4-((1S)-1,2-dihydroxyethyl)phenyl)ethynyl)benzoyl)(methyl)amino)-N-hydroxy-N',2-dimethylmalonamide and (2) an organic solvent, in the absence of water, wherein the organic solvent is one or two or more selected from aliphatic hydrocarbons, alcohols, ethers, ketones, esters, sulfoxides, nitriles, and amides.

**10.** The production method according to claim 9, wherein the aliphatic hydrocarbon is heptane, the alcohols are methanol, ethanol and 2-propanol, the ether is tetrahydrofuran, the ketone is acetone, the ester is ethyl acetate, the sulfoxide is dimethyl sulfoxide, the nitrile is acetonitrile, and the amide is dimethylacetamide.

**11.** The production method according to claim 9 or 10, wherein (2S)-2-((4-((4-((1S)-1,2-dihydroxyethyl)phenyl)ethynyl)benzoyl)(methyl)amino)-N-hydroxy-N',2-dimethylmalonamide is the crystal according to claim 1 or 2.

[Figure 1]

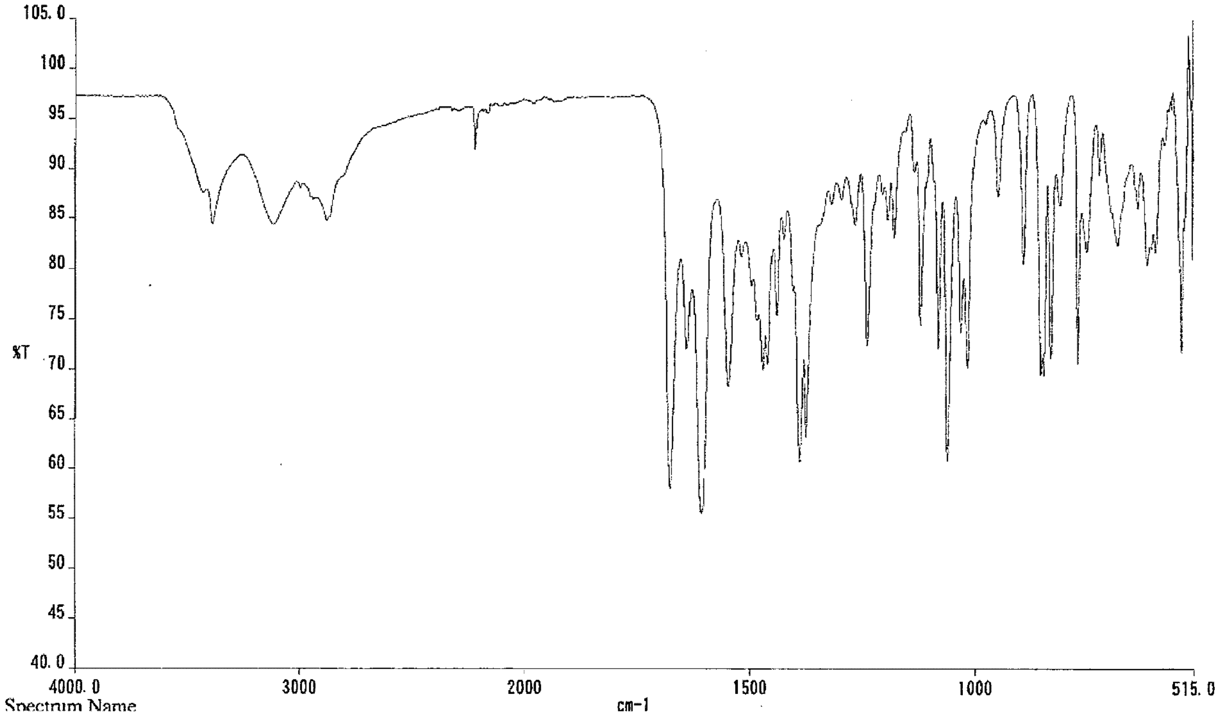


[Figure 2]

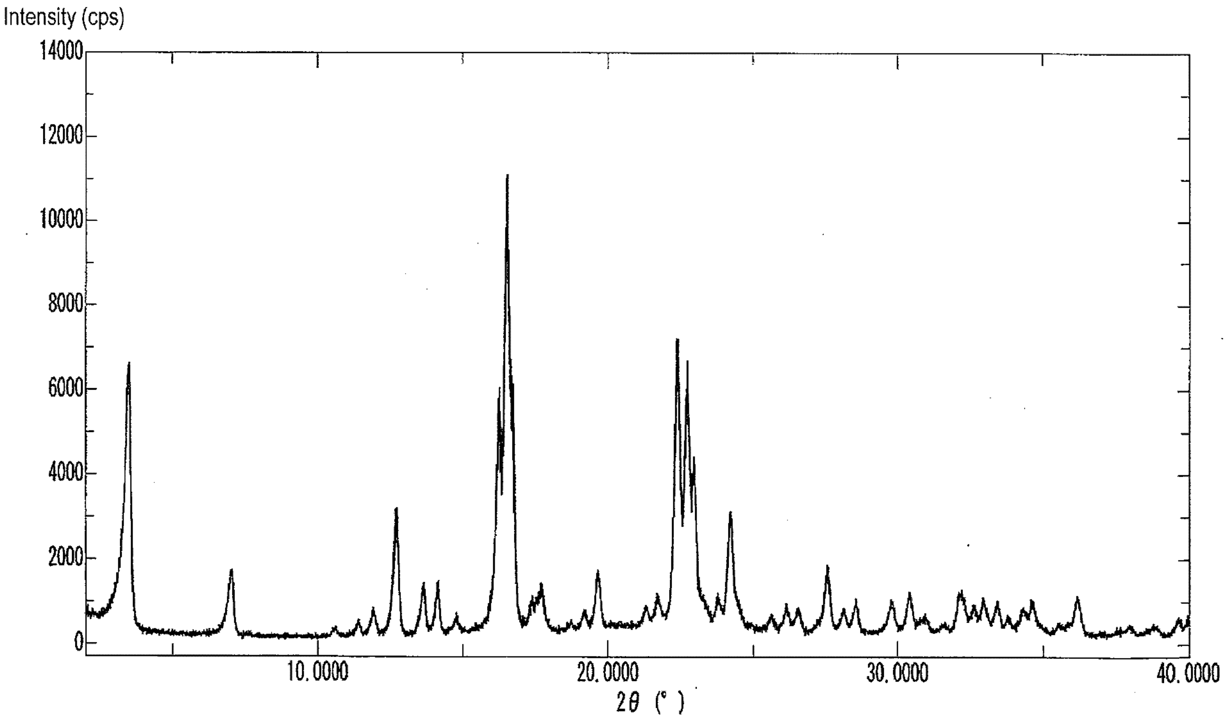

[Figure 3]

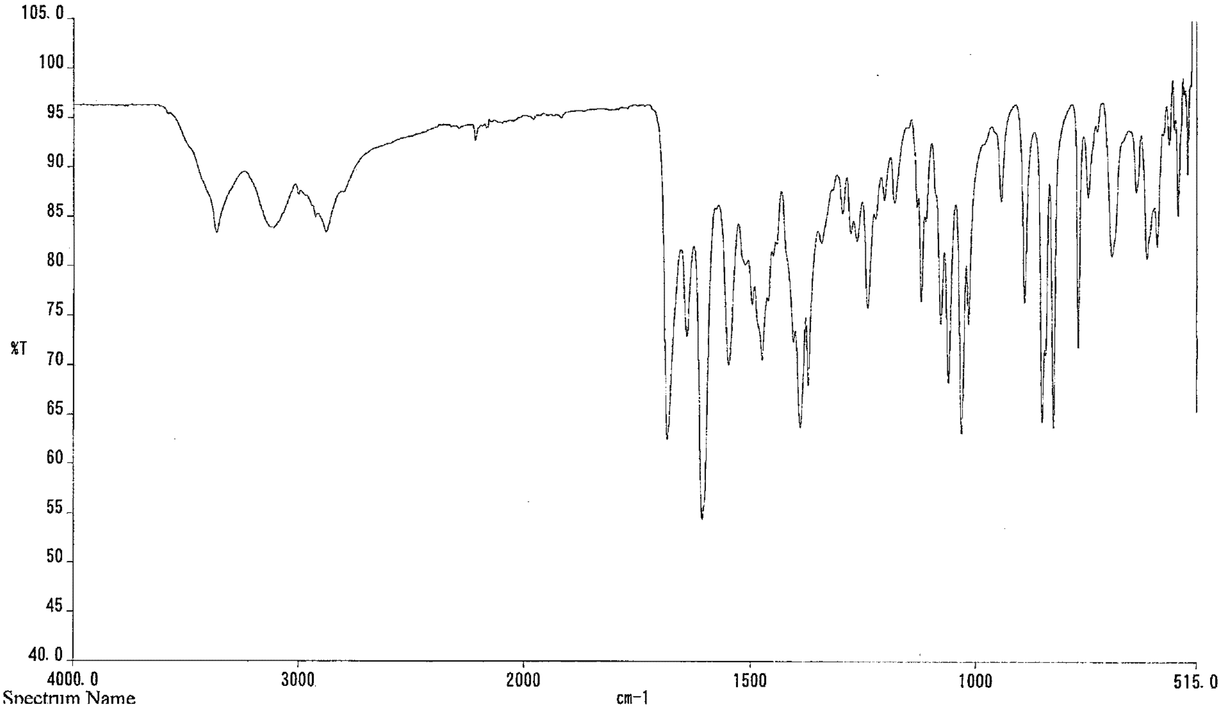


[Figure 4]

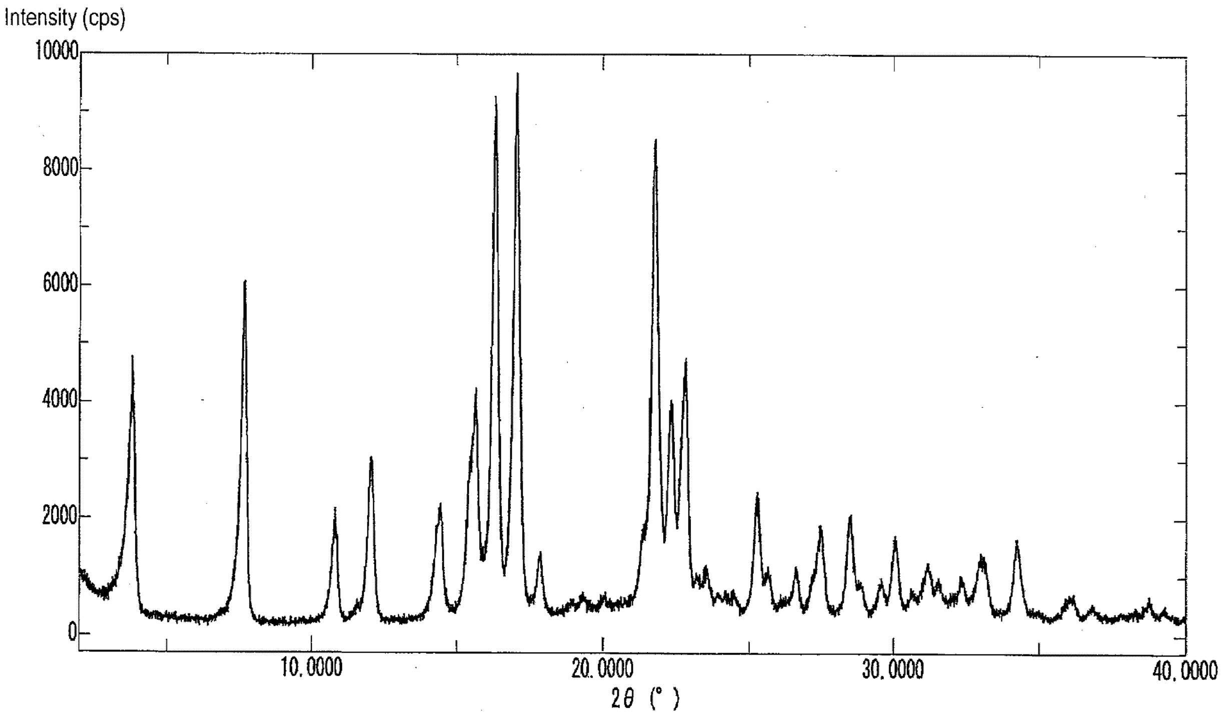

[Figure 5]

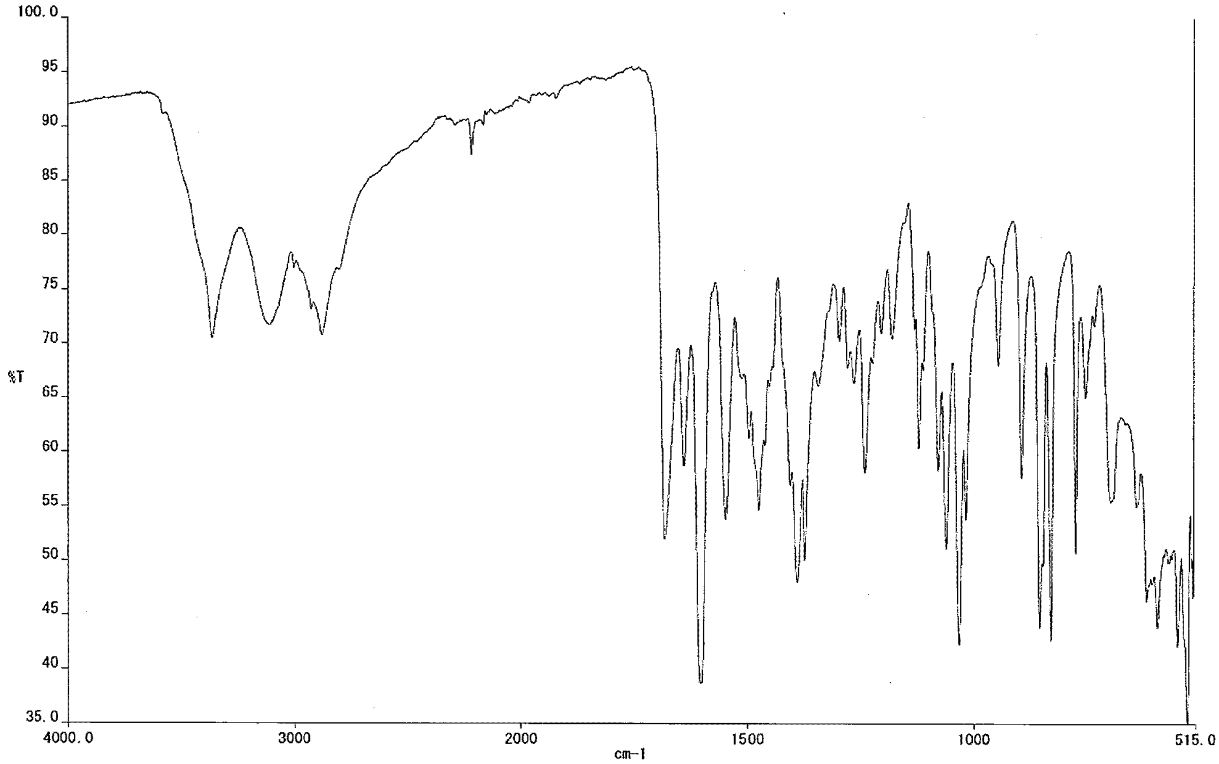


[Figure 6]

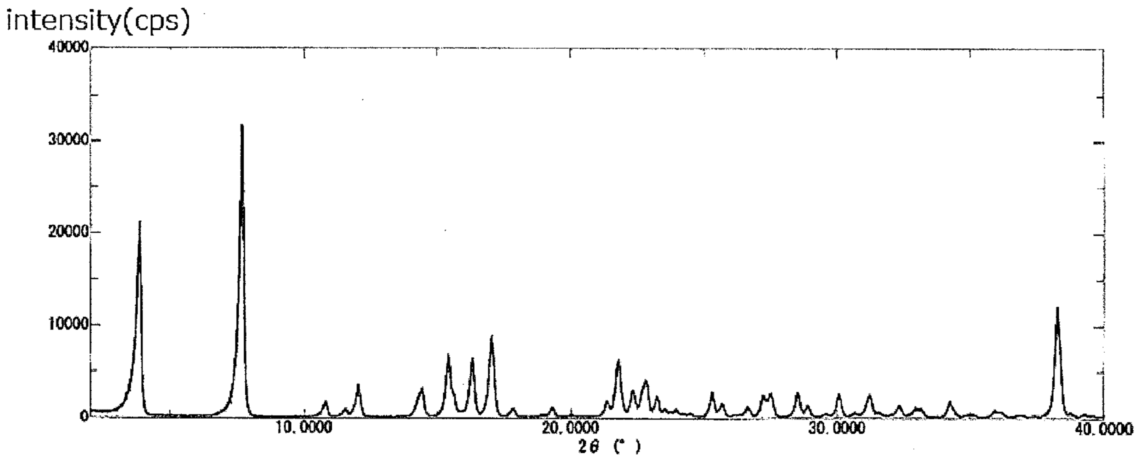

[Figure 7]

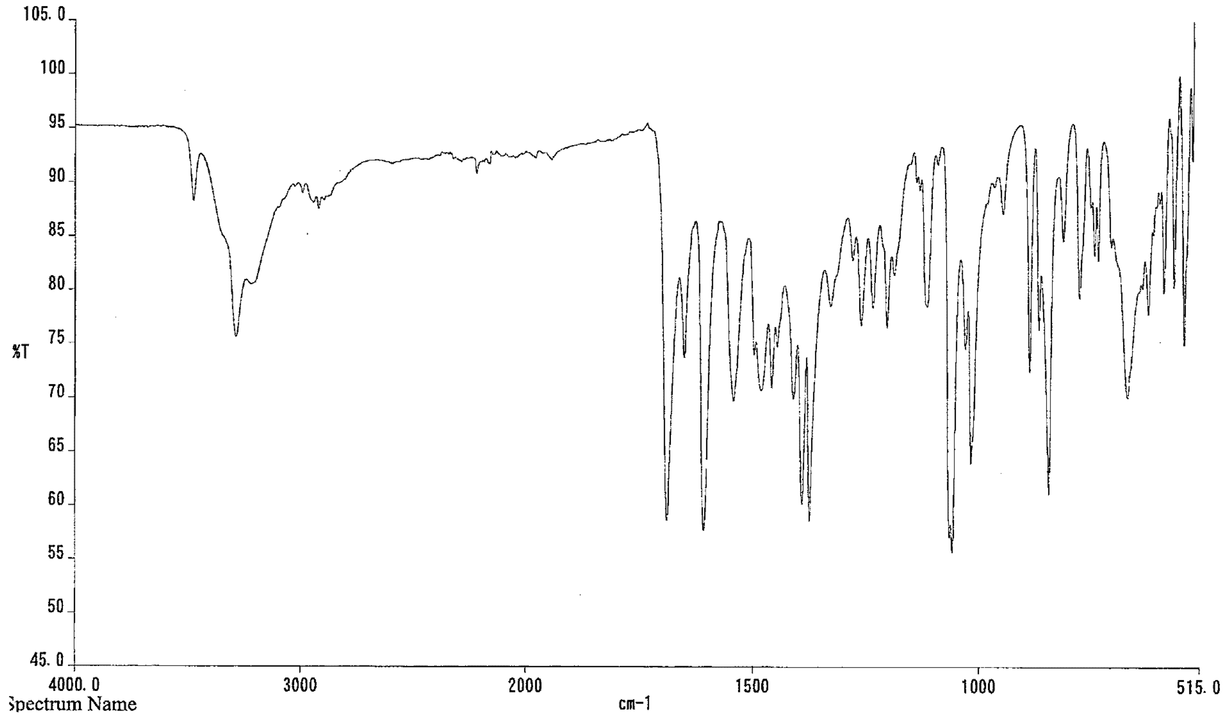


[Figure 8]

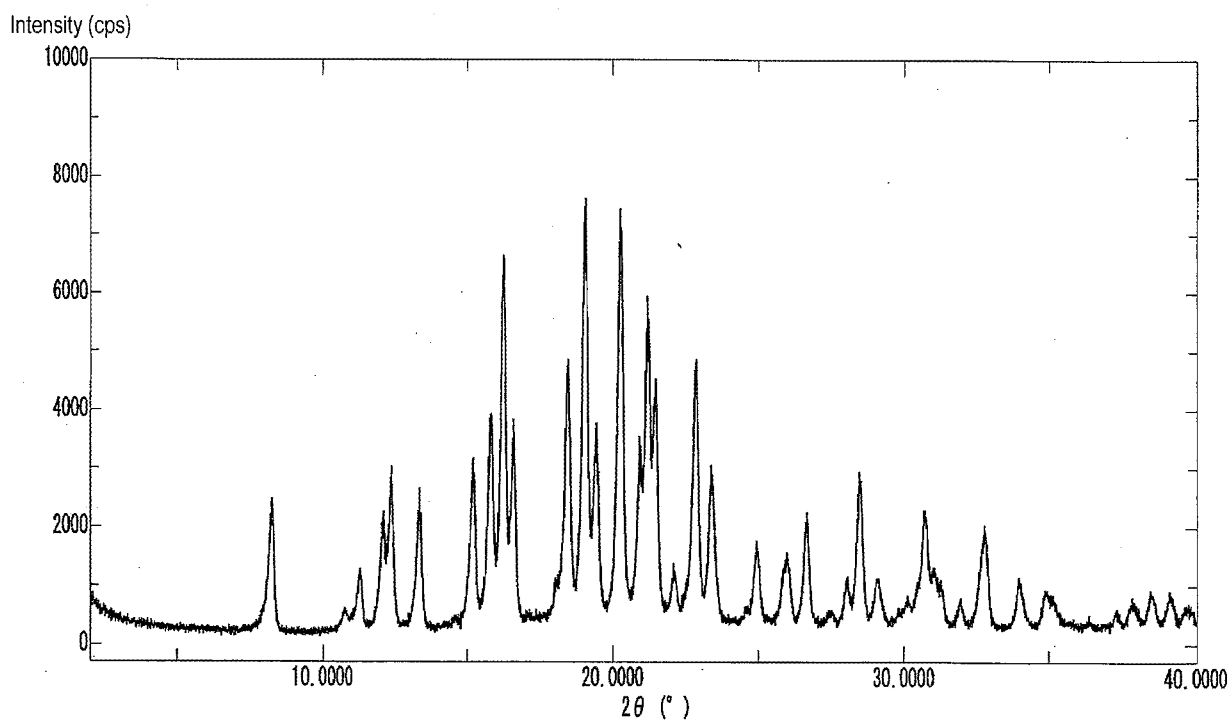

[Figure 9]

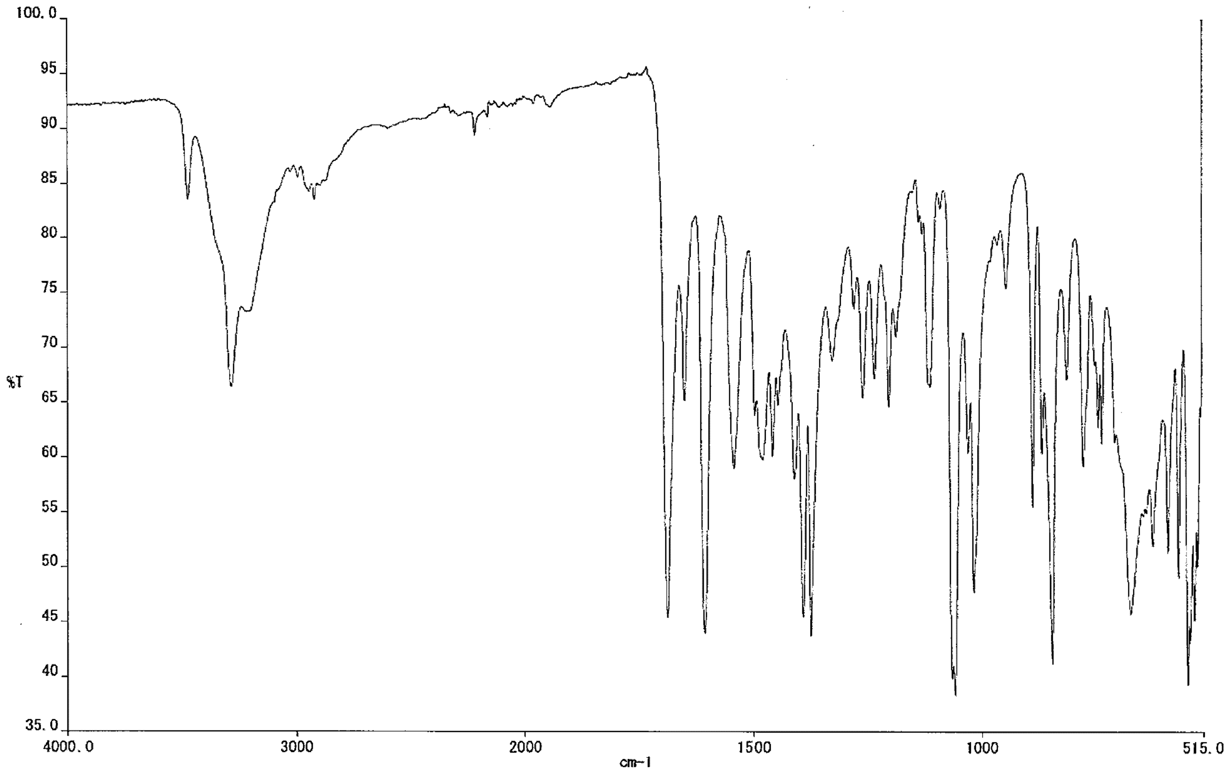


[Figure 10]

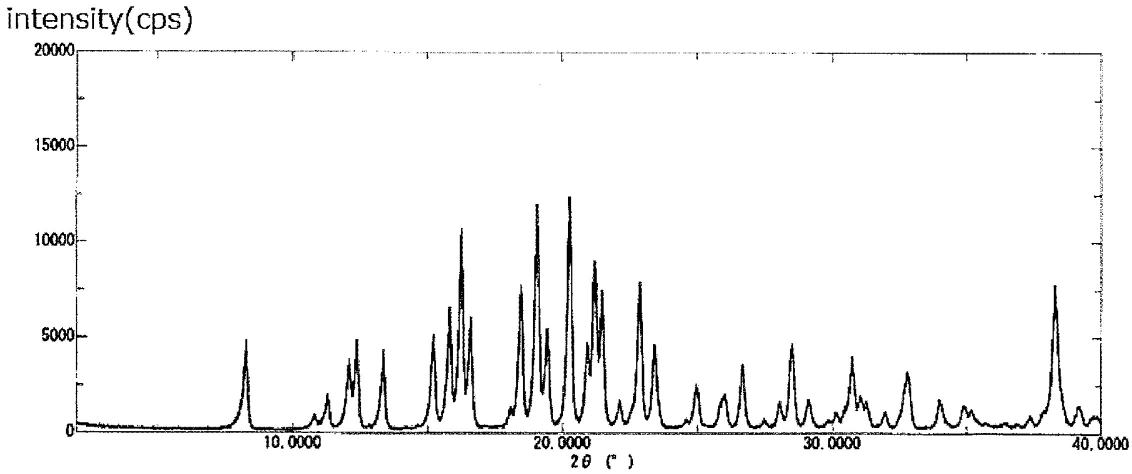

# INTERNATIONAL SEARCH REPORT

International application No. PCT/JP2017/013290

A. CLASSIFICATION OF SUBJECT MATTER
*C07C239/08*(2006.01)i, *A61K31/166*(2006.01)i, *A61P31/04*(2006.01)i, *A61P43/00*(2006.01)i

According to International Patent Classification (IPC) or to both national classification and IPC

B. FIELDS SEARCHED

Minimum documentation searched (classification system followed by classification symbols)
C07C239/08, A61K31/166, A61P31/04, A61P43/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | WO 2014/142298 A1 (Toyama Chemical Co., Ltd.), 18 September 2014 (18.09.2014), claims; example 20; test examples; paragraph [0058] & US 2016/0039751 A1 claims; example 20; test examples; paragraph [0135] & EP 2975022 A1 | 1-11 |
| X | WO 2016/039432 A1 (Toyama Chemical Co., Ltd.), 17 March 2016 (17.03.2016), claims; preparation example 5; paragraph [0014] (Family: none) | 1-11 |

☒ Further documents are listed in the continuation of Box C. ☐ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 01 May 2017 (01.05.17) | 13 June 2017 (13.06.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No.
PCT/JP2017/013290

C (Continuation). DOCUMENTS CONSIDERED TO BE RELEVANT

| Category* | Citation of document, with indication, where appropriate, of the relevant passages | Relevant to claim No. |
|---|---|---|
| X | WO 2016/039433 A1 (Toyama Chemical Co., Ltd.), 17 March 2016 (17.03.2016), claims; preparation example 5; paragraph [0015] (Family: none) | 1-11 |
| A | Yusaku SHIOJI, Kokei Seizai no Seizo Gijutsu, CMC Publishing Co., Ltd., 27 January 2003 (27.01.2003), page 12, '1.2.4 Kessho Takei' | 1-11 |
| A | edited by Noriaki HIRAYAMA, Yuki Kagobutsu Kessho Sakusei Handbook -Genri to Know-how-, Maruzen Co., Ltd., 25 July 2008 (25.07.2008), pages 57 to 58, '4.1 Introduction', pages 78 to 79, '4.5.1 Ippanteki na Kesshoka Joken' | 1-11 |
| A | Shozo ASAHARA, Yozai Handbook, Kodansha Ltd., 1985, pages 47 to 51, tables 3.3, 3.4 | 1-11 |
| P,X | JP 2017-14199 A (Toyama Chemical Co., Ltd.), 19 January 2017 (19.01.2017), claims; preparation example 3; paragraph [0031] (Family: none) | 1-11 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014142298 A